# EUROPEAN PATENT SPECIFICATION

(11) **EP 2 406 280 B1**
(45) Date of publication and mention of the grant of the patent: **14.05.2014**
(21) Application number: 10722160.8
(22) Date of filing: 15.03.2010
(51) Int. Cl.: C07K 14/47, C07K 14/78, C07K 16/18, C12N 15/113

(54) **BIOLOGICAL MATERIALS AND USES THEREOF**
BIOLOGISCHE MATERIALIEN UND VERWENDUNGEN DAVON
MATÉRIELS BIOLOGIQUES ET LEURS UTILISATIONS

(30) Priority: 13.03.2009 GB 0904355
(43) Date of publication of application: 18.01.2012
(60) Divisional application: 14162944.4
(73) Proprietor: Imperial Innovations Limited, South Kensington London SW7 2PG (GB)
(72) Inventor: MIDWOOD, Kim, Suzanne, Oxford OX3 7FY (GB); FOXWELL, Brian Maurice John, deceased (GB)
(74) Representative: Potter Clarkson LLP
(86) International application number: PCT/GB2010/000460
(87) International publication number: WO 2010/103289

(56) References cited:
- WO-A1-2008/154008
- WO-A2-2005/009366
- NAKAHARA HIROKI ET AL: "Deficiency of tenascin C attenuates allergen-induced bronchial asthma in the mouse." EUROPEAN JOURNAL OF IMMUNOLOGY DEC 2006 LNKD- PUBMED:17125141, vol. 36, no. 12, December 2006 (2006-12), pages 3334-3345, XP002594137 ISSN: 0014-2980
- CHIQUET-EHRISMANN RUTH ET AL: "Tenascins: regulation and putative functions during pathological stress." THE JOURNAL OF PATHOLOGY JUL 2003 LNKD- PUBMED:12845616, vol. 200, no. 4, July 2003 (2003-07), pages 488-499, XP002594134 ISSN: 0022-3417 cited in the application
- TAVAZOIE SOHAIL F ET AL: "Endogenous human microRNAs that suppress breast cancer metastasis." NATURE 10 JAN 2008 LNKD- PUBMED:18185580, vol. 451, no. 7175, 10 January 2008 (2008-01-10), pages 147-152, XP002594135 ISSN: 1476-4687
- CALVO A ET AL: "Identification of VEGF-regulated genes associated with increased lung metastatic potential: functional involvement of tenascin-C in tumor growth and lung metastasis." ONCOGENE 11 SEP 2008 LNKD- PUBMED:18504437, vol. 27, no. 40, 11 September 2008 (2008-09-11), pages 5373-5384, XP002594136 ISSN: 1476-5594
- MIDWOOD KIM ET AL: "Tenascin-C is an endogenous activator of Toll-like receptor 4 that is essential for maintaining inflammation in arthritic joint disease." NATURE MEDICINE JUL 2009 LNKD- PUBMED:19561617, vol. 15, no. 7, July 2009 (2009-07), pages 774-780, XP002594138 ISSN: 1546-170X cited in the application

## Description

The present invention relates to tenascin-C and its activity in chronic inflammation. There is also provided modulators of tenascin-C and its biological activity.

Inflammation is the complex biological response of tissues to harmful stimuli, such as pathogens, tissue damage, or irritants. It is a protective attempt by the tissue to remove the injurious stimuli as well as initiate the healing process for the tissue. Abnormalities associated with inflammation comprise a large, unrelated group of disorders which underlie a variety of human diseases (inflammatory disorders). Examples of diseases with an inflammatory aspect include (but are not limited to) asthma, autoimmune disease, glomerulonephritis, allergy (hypersensitivities), inflammatory bowel diseases, reperfusion injury, rheumatoid arthritis and transplant rejection.

In particular, chronic inflammation is a debilitating and serious condition associated with many of the above diseases and is characterised by persistent inflammation at a site of infection or injury, or in relation to altered immune responses such as in autoimmune disease.

Rheumatoid arthritis (RA) is a typical example of, though by no means the only, a chronic inflammatory condition. RA is characterized by synovial inflammation and destruction of joint cartilage and bone mediated by persistent synthesis of pro-inflammatory cytokines and matrix metalloproteinases (MMPs). Biological compounds that suppress the synthesis of inflammatory cytokines such as TNFα and IL-6 are successful at treating RA in the short-term. However, repeated treatments are required, which renders this an expensive therapeutic approach, and does not provide long-term remission. Furthermore, total systemic suppression of cytokine function is not without inherent problems such as increased infectious risk. Thus, despite advances in care, there remains an unmet need for an economical mode of treatment of chronic inflammatory that is efficacious over the long term (Smolen (2006) and Williams (2007)).

The mechanisms that underpin disease chronicity remain unclear and the factor(s) that drive the prolonged expression of inflammatory and destructive mediators are currently unknown.

Toll-like receptors (TLRs) play a key role in driving the production of inflammatory mediators in RA and blockade of TLR function may be of significant clinical benefit (reviewed in Brentano (2005) and O'Neill (2002)). This receptor family forms an integral part of the immune system. TLRs mediate host defence against infection and injury by recognising both pathogen-associated molecular patterns (PAMPs) and damage-associated molecular patterns (DAMPs) (Matzinger (2002)). DAMPs are endogenous pro-inflammatory molecules generated upon tissue injury and include intracellular molecules released from damaged or necrotic cells, fragments of extracellular matrix (ECM) molecules or ECM molecules up regulated upon injury (reviewed in Bianchi (2007) and Gordon (2002)).

Upon activation, TLRs promote both innate and adaptive immune responses including stimulation of expression of pro-inflammatory cytokines and MMPs (Medzhitov (2002)). TLRs are expressed at high levels in synovial tissue from RA patients (Radstake (2004), Roelofs (2005), Sacre (2007), and (Sacre, manuscript submitted 2008) and mice with targeted deletions or loss of function mutations in TLR4 are protected from experimental arthritis (Choe (2003) and Lee (2005). Furthermore, inhibitors of TLR4 can reduce destructive arthritis in mice (Abdollahi-Roodsaz (2007)) and a putative TLR4 inhibitor improved symptoms in 15 out of 23 patients with moderate to severe RA in a preliminary phase I trial (Vanags (2006). However, it is unclear which TLR ligand(s) are involved in disease pathogenesis.

Tenascin-C is an ECM glycoprotein that is associated with tissue injury and wound repair. Tenascin-C is expressed specifically at during active tissue remodelling during embryogenesis, being first observed during gastrulation and somite formation. In later stages of development expression is restricted to sites of branching morphogenesis of mammary gland and the lung, in the developing skeleton, cardiovascular system and in connective tissues at sites of epithelial to mesenchymal transformation. Expression is down regulated once these processes cease and before embryogenesis is complete (Jones (2000)).

Tenascin-C is not normally expressed in healthy adult tissue but, in adults, is specifically and transiently up-regulated during acute inflammation and persistently expressed in chronic inflammation (reviewed in Chiquet-Ehrismann (2003)). Immunohistochemical studies show that little tenascin-C is expressed in normal human joints but levels are greatly increased in RA synovia, in areas of inflammation and fibrosis, specifically below the synovial lining, in the invading pannus and around blood vessels (Cutolo (1992), MacCachren (1992) and Salter (1993)). There is also a significant increase in tenascin-C levels in synovial fluid from RA patients (Chevalier (1994) and Hasegawa (2007)) and in RA cartilage (Salter (1993) and Chevalier (1994)).

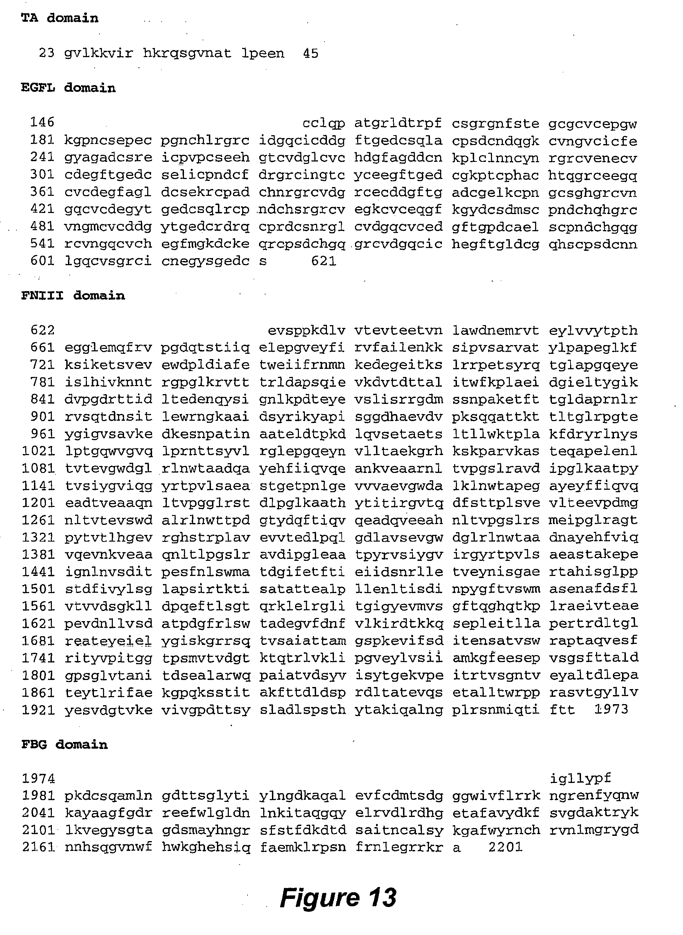

Tenascin-C is a large hexameric protein of 1.5 million Da. Each chain comprises different domains, including an assembly domain (TA), EGF-like repeats (EGF-L), fibronectin type III-like repeats (TNIII) and a fibrinogen-like globe (FBG) (reviewed in Orend (2005)). The sequences of tenascin-C and its domains are shown in Figure 13.

Previously, the role of tenascin-C in inflammation has been uncertain, with evidence showing varying effects on different immune cells. For example tenascin-C has been shown to supports primary human peripheral blood and tonsillar lymphocyte adhesion and rolling, thereby suggesting a role in stimulating lymphocyte migration (Clark (1997)). In addition, tenascin-C null mice exhibit reduced lymphocyte infiltration and lower levels of IFN, TNF and IL-4 mRNA upon concanavalin A-induced liver injury in mice (El-Karef (2007)). Thus, evidence suggests tenascin-C is involved in promoting activity of acute inflammatory cells. However, tenascin-C has also been reported to inhibit monocyte chemotaxis in vitro (Loike (2001)) and tenascin-C-null mice exhibit increased migration of monocytes and macrophages in mammary tumour stroma (Talts (1999)). This evidence therefore suggests tenascin-C has a role in inhibition of inflammatory cells.

The inventors have shown that tenascin-C is an endogenous TLR4 ligand that it is required for destructive joint inflammation observed in arthritis.

Furthermore, it is now shown that tenascin-C is not involved with induction of inflammation (acute inflammatory response) but instead is involved in the prolonging of the inflammatory response characterising the chronic inflammatory condition. In particular, tenascin-C has now been shown to be an endogenous activator of TLR4 and demonstrated that this molecule is required for destructive joint inflammation.

A role for tenascin-C in mediating an immune response in the joint was demonstrated by induction of joint inflammation upon intra-articular injection of the FBG domain of tenascin-C in mice *in vivo.* Moreover, acute joint inflammation induced by zymosan was not as prolonged in tenascin-C deficient mice. Both the wild type and tenascin-C null mice responded to acute inflammation induction by zymosan equally, demonstrating that tenascin-C does not appear to be involved in the initiation of inflammation. However, the less persistent synovitis exhibited by tenascin-C null mice indicates a role in the maintenance of joint inflammation. The importance of tenascin-C in prolonging joint inflammation was underscored by the observation that targeted deletion of tenascin-C protected mice from sustained erosive joint inflammation during arthritis induced by immunization with mBSA.

Tenascin-C has now been shown to be capable of activating cells in the joint and the primary active domain of tenascin-C has been mapped to the fibrinogen-like globe (FBG), a 227 amino acid (26.9 kDa) globular domain at the C terminal of the molecule (Siri (1991)).

Addition of FBG to synovial membrane cultures from RA patients enhanced the spontaneous release of pro-inflammatory cytokines. It also stimulated synthesis of TNF-α, IL-6 and IL-8 in primary human macrophages and IL-6 in RA synovial fibroblasts via activation of TLR4 and MyD88 dependent signalling pathways.

It has now been shown that, as in the case of LPS, TLR4 expression is necessary for induction of cytokine synthesis by FBG. However, unlike LPS, neither CD14 nor MD-2 appears to be required for TLR-4 activation. CD14 is dispensable for activation of TLR4 by other ligands. It is not required for TLR4 to respond to lipid A in a MyD88 dependent manner (Jiang (2005)), fibronectin EDA can activate mast cells even in the absence of CD14 (Gondokaryono (2007)) and hyaluronic acid activation of human monocytic THP-1 cells requires a complex of TLR4, CD44 and MD-2, but not CD14 (Taylor (2007)).

Formation of distinct receptor complexes by each TLR4 ligand may facilitate recruitment of different intracellular adapter/signalling molecules. This may account for the differential cellular responses we observe with FBG and LPS, for example lack of IL-8 induction by FBG in RA synovial fibroblasts. Similarly, hyaluronic acid activation of the TLR4 and CD44 complex induces a pattern of gene expression in mouse alveolar macrophage cell lines that is different to LPS (Taylor (2007)). That FBG induces IL-8 synthesis in human macrophages, suggests cell type specific ligand recognition and/or signalling occurs.

The tightly regulated pattern of expression of tenascin-C makes it an attractive target for treating chronic inflammation. It is predominantly absent from healthy adults, however expression is specifically induced upon tissue injury. During acute inflammation tenascin-C is transiently expressed: induction often precedes inflammation and both mRNA and protein are absent from the tissue by the time inflammation is resolved (reviewed In Chiquet-Ehrismann (2003)).

Persistent expression of tenascin-C has now been shown to be associated with chronic inflammation. In addition to RA, increased tenascin-C levels are observed in other autoimmune diseases including multiple sclerosis (Gutowski (1999)) and Sjogrens disease (Amin (2001)), and in non-healing wounds and diabetic and venous ulcers (Loots (1998)). *De novo* synthesis of tenascin-C correlates well with the intensity of inflammation in diseases of the oral mucosa and plasma levels of tenascin-C are a reliable indicator for the activity of inflammatory bowel diseases before and after medication or surgery (reviewed in Chiquet-Ehrismann (2003)).

In a first aspect of the invention there is provided an agent for modulation of a chronic inflammatory response wherein the agent modulates the biological activity of tenascin-C, wherein the agent is an antibody or antigen-binding fragment thereof having specificity for the FBG domain of tenascin-C.

The agent of the first aspect of the invention may modulate the biological activity of tenascin-C by altering binding properties of tenascin-C.

Also described herein are agents which may modulate the biological activity of tenascin-C by altering the transcription and/or translation of tenascin-C.

Such agents may be identified using methods well known in the art, such as:
(a) by determining the effect of a test agent on levels of expression of tenascin-C, for example by Southern blotting or related hybridisation techniques;
(b) by determining the effect of a test agent on levels of tenascin-C protein, for example by immunoassays using anti- tenascin-C antibodies; and
(c) by determining the effect of a test agent on a functional marker or result of tenascin-C activity, for example via the methods of the examples.

Agents disclosed herein may down-regulate the biological activity of tenascin-C.

Agents disclosed herein may up-regulate the biological activity of tenascin-C. The desirability of up-regulating activity of immune and inflammatory molecules and cells is relevant to the production of therapies for compromised immune and inflammatory patients and in the development of vaccines. (see Harandi (2009)).

The agent described herein may be an inhibitor of transcription of tenascin-C.

The agent described herein may be an inhibitor of translation of tenascin-C.

The agent of the first aspect of the invention may be an inhibitor of the binding properties of tenascin-C. For example, the agent may alter the conformation of tenascin-C such that it is no longer able to bind to its receptor.

The agent of the first aspect of the invention may be a competitive binding inhibitor of tenascin-C. It will be appreciated by persons skilled in the art that the agent may also inhibit the biological activity of tenascin-C by blocking tenascin-C receptor function either directly (by acting as an tenascin-C receptor antagonist) or indirectly (by binding intermediary or assisting molecules).

The agent of the first aspect of the invention may be an antagonist of the TLR-4 receptor.

It will be appreciated by persons skilled in the art that inhibition of the biological activity of tenascin-C by an agent of the invention may be in whole or in part. For example, the agent may inhibit the biological activity of tenascin-C by at least 10%, preferably at least 20%, 30%, 40%, 50%, 60%, 70%, 80% or 90%, and most preferably by 100% compared to the biological activity of tenascin-C on inflammatory cells which have not been exposed to the agent.

The agent described herein may be selected from the group consisting of short interfering RNA (SiRNA) molecules, short hairpin RNA molecules (shRNA), antisense oligonucleotides, compounds with binding affinity for tenascin-C, antibodies (polyclonal or monoclonal) and antigen-binding fragments thereof, small inhibitor compounds, polypeptides and proteins.

In one example the agent is an siRNA. RNA interference is a two-step process. The first step, which is termed as the initiation step, input dsRNA is digested into 21-23 nucleotide (nt) small interfering RNAs (siRNA), probably by the action of Dicer, a member of the Rnase III family of dsRNA-specific ribonucleases, which processes (cleaves) dsRNA (introduced directly or via a transgene or a virus) in an ATP-dependent manner. Successive cleavage events degrade the RNA to 19-21 bp duplexes (siRNA) each with 2-nucleotide 3' overhangs (Hutvagner & Zamore, 2002, Curr. Opin. Genetics and Development 12:225-232; Bernstein, 2001, Nature 409:363-366).

In the effector step, the siRNA duplexes bind to a nuclease complex to form the RNA-induced silencing complex (RISC). An ATP-dependent unwinding of the siRNA duplex is required for activation of the RISC. The active RISC then targets the homologous transcript by base pairing interactions and cleaves the mRNA into 12 nucleotide fragments from the 3' terminus of the siRNA (Hutvagner & Zamore, 2002, supra.; Hammond et al., 2001, Nat. Rev. Gen. 2:110-119 (2001); Sharp, 2001, Genes. Dev. 15:485-90). Although the mechanism of cleavage is still to be elucidated, research indicates that each RISC contains a single siRNA and an RNase (Hutvagner & Zamore, 2002, supra.).

In view of the remarkable potency of RNAi, an amplification step within the RNAi pathway has been suggested. Amplification could occur by copying of the input dsRNAs which would generate more siRNAs, or by replication of the siRNAs formed. Alternatively, or additionally, amplification could be effected by multiple turnover events of the RISC (Hammond et al., 2001, supra.; Hutvagner & Zamore, 2002, supra.). Additional information on RNAi can be found in the following reviews, Tuschl, 2001, Chem. Biochem. 2:239-245, Cullen, 2002, Nat. Immunol. 3:597-599 and Brantl, 2002, Biochem. Biophys Act. 1575:15-25.

Synthesis of RNAi molecules suitable for use with the present invention can be effected as follows. First, the tenascin-C mRNA sequence is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex (Tuschl, ChemBiochem. 2:239-245). It will be appreciated, however, that siRNAs directed at untranslated regions may also be effective.

Second, potential target sites are compared to an appropriate genomic database (e.g. human, mouse, rat, etc.) using sequence alignment software, such as the BLAST (www.ncbi.nlm.nih.gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55%. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

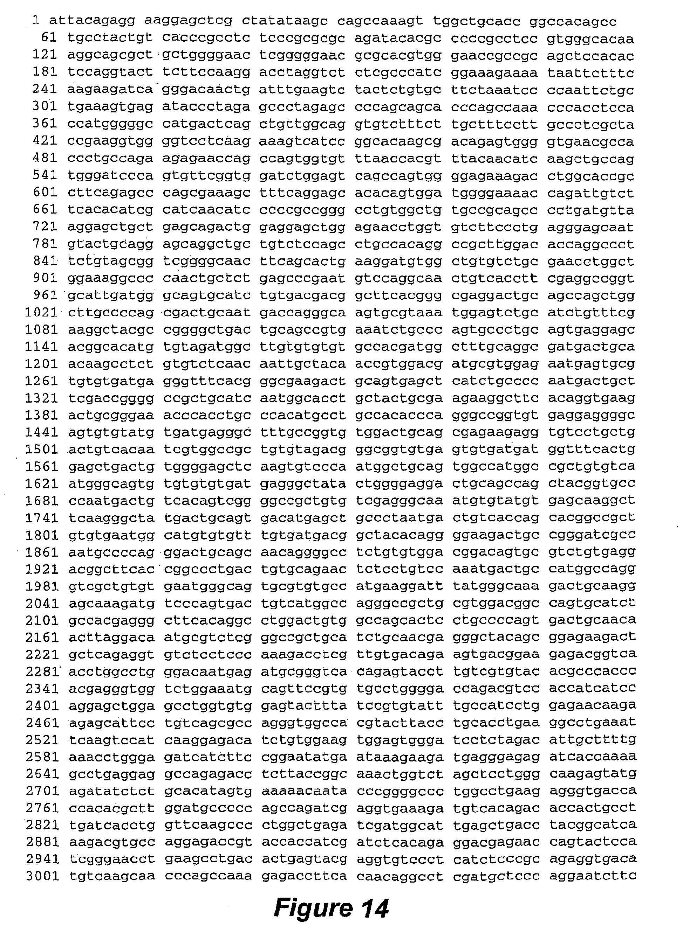

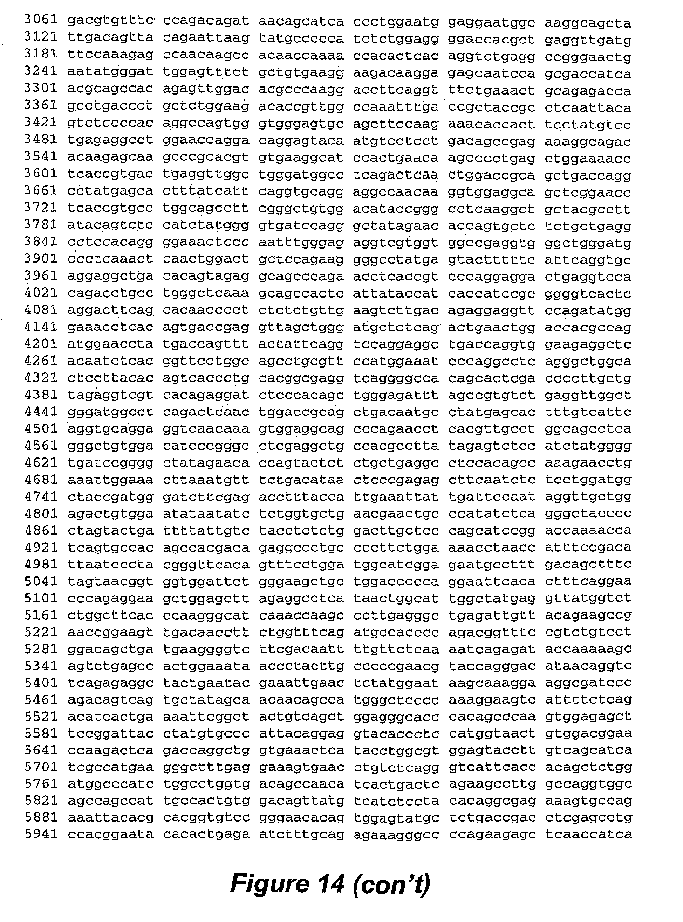

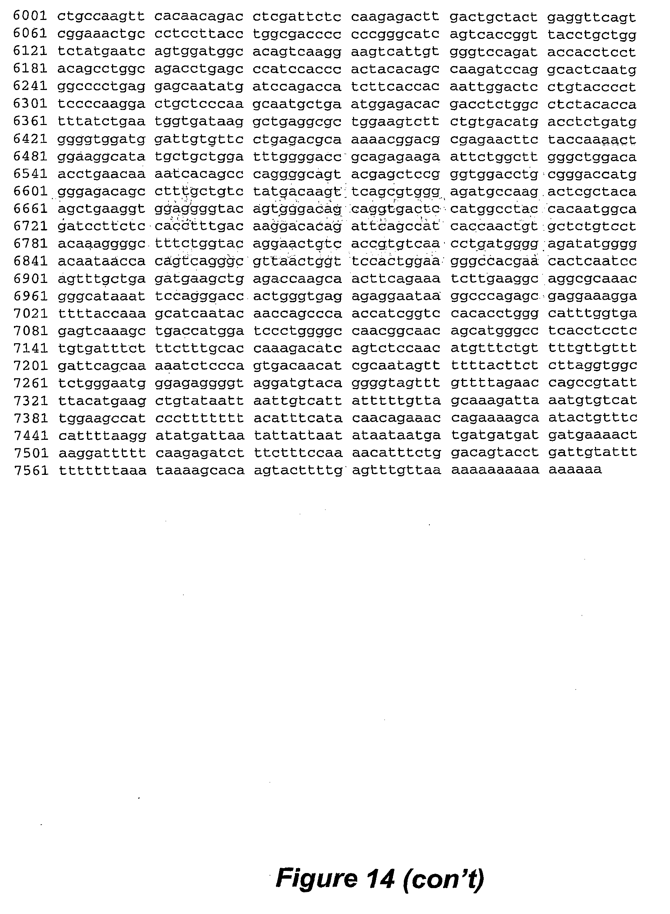

Suitable SiRNA molecules can be synthesised as described above such that they are complementary and therefore bind to the whole nucleotide sequence of tenascin-C or portions thereof. The nucleotide sequence of tenascin-C is found in figure 14.

In one example the agent may be a short hairpin RNA (ShRNA).

A small hairpin RNA or short hairpin RNA (shRNA) is a sequence of RNA that makes a tight hairpin turn that can be used to silence gene expression via RNA interference. shRNA uses a vector (typically adenovirus or lentivirus) introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that it is bound to it. (McIntyre (2006) and Paddison (2002))

The agent of the first aspect of the invention may be a domain of tenascin-C or variant thereof. The FBG domain has been shown to be predominantly involved in the interaction of tenascin-C with its target in relation to the persistence of chronic inflammation. Accordingly the preferred domain is the FBG domain (sequence shown in figure 13) or variants thereof.

In an alternative example, the agent is an antisense oligonucleotide.

The design of antisense molecules which can be used to decrease efficiently tenascin-C levels/activity requires consideration of two aspects important to the antisense approach. The first aspect is delivery of the oligonucleotide into the cytoplasm of the cancer cells, while the second aspect is design of an oligonucleotide which specifically binds the designated mRNA within cells in a way which inhibits translation thereof.

The prior art teaches a number of delivery strategies which can be used to efficiently deliver oligonucleotides into a wide variety of cell types (for example, see Luft, 1998, J Mol Med 76:75-6; Kronenwett et al., 1998, Blood 91:852-62; Rajur et al., 1997, Bioconjug Chem 8:935-40; Lavigne et al., 1997, Biochem Biophys Res Commun 237:566-71; Aoki et al., 1997, Biochem Biophys Res Commun 231:540-5).

In addition, algorithms for identifying those sequences with the highest predicted binding affinity for their target mRNA based on a thermodynamic cycle that accounts for the energetics of structural alternations in both the target mRNA and the oligonucleotide are available (for example, see Walton et al., 1999, Biotechnol Bioeng 65:1-9).

Several approaches for designing and predicting efficiency of specific oligonucleotides using an *in vitro* system are also known (for example, see Matveeva et al., 1998, Nature biotechnology 16:1374-1375).

Several clinical trails have demonstrated safety, feasibility and activity of antisense oligonucleotides. For example, antisense oligonucleotides suitable for the treatment of cancer have been successfully used (Holmlund et al., 1999, Curr Opin Mol Ther 1:372-85; Gerwitz, 1999, Curr Opin Mol Ther 1:297-306). More recently, antisense-mediated suppression of human heparanase gene expression has been reported to inhibit pleural dissemination of human cancer cells in a mouse model (Uno et al., 2001, Cancer Res 61:7855-60).

Thus, persons skilled in the art are readily able to design and implement antisense approaches suitable for modulating expression of tenascin-C.

Advantageously, the antisense oligonucleotide is 15 to 35 bases in length. For example, 20-mer oligonucleotides have been shown to inhibit the expression of the epidermal growth factor receptor mRNA (Witters et al, Breast Cancer Res Treat 53:41-50 (1999)) and 25-mer oligonucleotides have been shown to decrease the expression of adrenocorticotropic hormone by greater than 90% (Frankel et al, J Neurosurg 91:261-7 (1999)). However, it is appreciated that it may be desirable to use oligonucleotides with lengths outside this range, for example 10, 11, 12, 13, or 14 bases, or 36, 37, 38, 39 or 40 bases.

It will be further appreciated by person skilled in the art that oligonucleotides are subject to being degraded or inactivated by cellular endogenous nucleases. To counter this problem, it is possible to use modified oligonucleotides, *e.g.* having altered internucleotide linkages, in which the naturally occurring phosphodiester linkages have been replaced with another linkage. For example, Agrawal et al (1988) Proc. Natl. Acad. Sci. USA 85, 7079-7083 showed increased inhibition in tissue culture of HIV-1 using oligonucleotide phosphoramidates and phosphorothioates. Sarin et al (1988) Proc. Natl. Acad. Sci. USA 85, 7448-7451 demonstrated increased inhibition of HIV-1 using oligonucleotide methylphosphonates. Agrawal et al (1989) Proc. Natl. Acad. Sci. USA 86, 7790-7794 showed inhibition of HIV-1 replication in both early-infected and chronically infected cell cultures, using nucleotide sequence-specific oligonucleotide phosphorothioates. Leither et al (1990) Proc. Natl. Acad. Sci. USA 87, 3430-3434 report inhibition in tissue culture of influenza virus replication by oligonucleotide phosphorothioates.

Oligonucleotides having artificial linkages have been shown to be resistant to degradation *in vivo*. For example, Shaw et al (1991) in Nucleic Acids Res. 19, 747-750, report that otherwise unmodified oligonucleotides become more resistant to nucleases *in vivo* when they are blocked at the 3' end by certain capping structures and that uncapped oligonucleotide phosphorothioates are not degraded *in vivo.*

A detailed description of the H-phosphonate approach to synthesising oligonucleoside phosphorothioates is provided in Agrawal and Tang (1990) Tetrahedron Letters 31, 7541-7544, the teachings of which are hereby incorporated herein by reference. Syntheses of oligonucleoside methylphosphonates, phosphorodithioates, phosphoramidates, phosphate esters, bridged phosphoramidates and bridge phosphorothioates are known in the art. See, for example, Agrawal and Goodchild (1987) Tetrahedron Letters 28, 3539; Nielsen et al (1988) Tetrahedron Letters 29, 2911; Jager et al (1988) Biochemistry 27, 7237; Uznanski et al (1987) Tetrahedron Letters 28, 3401; Bannwarth (1988) Helv. Chim. Acta. 71, 1517; Crosstick and Vyle (1989) Tetrahedron Letters 30, 4693; Agrawal et al (1990) Proc. Natl. Acad. Sci. USA 87, 1401-1405, the teachings of which are incorporated herein by reference. Other methods for synthesis or production also are possible. In a preferred embodiment the oligonucleotide is a deoxyribonucleic acid (DNA), although ribonucleic acid (RNA) sequences may also be synthesised and applied.

The oligonucleotides useful in the examples described herein preferably are designed to resist degradation by endogenous nucleolytic enzymes. *In vivo* degradation of oligonucleotides produces oligonucleotide breakdown products of reduced length. Such breakdown products are more likely to engage in non-specific hybridisation and are less likely to be effective, relative to their full-length counterparts. Thus, it is desirable to use oligonucleotides that are resistant to degradation in the body and which are able to reach the targeted cells. The present oligonucleotides can be rendered more resistant to degradation *in vivo* by substituting one or more internal artificial internucleotide linkages for the native phosphodiester linkages, for example, by replacing phosphate with sulphur in the linkage. Examples of linkages that may be used include phosphorothioates, methylphosphonates, sulphone, sulphate, ketyl, phosphorodithioates, various phosphoramidates, phosphate esters, bridged phosphorothioates and bridged phosphoramidates. Such examples are illustrative, rather than limiting, since other internucleotide linkages are well known in the art. The synthesis of oligonucleotides having one or more of these linkages substituted for the phosphodiester internucleotide linkages is well known in the art, including synthetic pathways for producing oligonucleotides having mixed internucleotide linkages.

Oligonucleotides can be made resistant to extension by endogenous enzymes by "capping" or incorporating similar groups on the 5' or 3' terminal nucleotides. A reagent for capping is commercially available as Amino-Link II^{™} from Applied BioSystems Inc, Foster City, CA. Methods for capping are described, for example, by Shaw et al (1991) Nucleic Acids Res. 19, 747-750 and Agrawal et al (1991) Proc. Natl. Acad. Sci. USA 88(17), 7595-7599.

A further method of making oligonucleotides resistant to nuclease attack is for them to be "self-stabilised" as described by Tang et al (1993) Nuc/. Acids Res. 21, 2729-2735. Self-stabilised oligonucleotides have hairpin loop structures at their 3' ends, and show increased resistance to degradation by snake venom phosphodiesterase, DNA polymerase I and foetal bovine serum. The self-stabilised region of the oligonucleotide does not interfere in hybridisation with complementary nucleic acids, and pharmacokinetic and stability studies in mice have shown increased *in vivo* persistence of self-stabilised oligonucleotides with respect to their linear counterparts.

In an example where the agent is a compound with binding affinity for tenascin-C, the compound may bind substantially reversibly or substantially irreversibly to an active site of tenascin-C. In a further example, the compound may bind to a portion of tenascin-C that is not the active site so as to interfere with the binding of the tenascin-C to a ligand or receptor. In a still further example, the compound may bind to a portion of tenascin-C so as to decrease the proteins activity by an allosteric effect. This allosteric effect may be an allosteric effect that is involved in the natural regulation of the activity ofTenascin-C, for example in the activation of the tenascin-Cby an "upstream activator".

Methods for detecting interactions between a test compound and tenascin-C are well known in the art. For example ultrafiltration with ion spray mass spectroscopy/HPLC methods or other physical and analytical methods may be used. In addition, Fluorescence Energy Resonance Transfer (FRET) methods may be used, in which binding of two fluorescent labelled entities may be measured by measuring the interaction of the fluorescent labels when in close proximity to each other.

Alternative methods of detecting binding of a polypeptide to macromolecules, for example DNA, RNA, proteins and phospholipids, include a surface plasmon resonance assay, for example as described in Plant et al., 1995, Analyt Biochem 226(2), 342-348. Methods may make use of a polypeptide that is labelled, for example with a radioactive or fluorescent label.

A further method of identifying a compound that is capable of binding to the polypeptide is one where the polypeptide is exposed to the compound and any binding of the compound to the said polypeptide is detected and/or measured. The binding constant for the binding of the compound to the polypeptide may be determined. Suitable methods for detecting and/or measuring (quantifying) the binding of a compound to a polypeptide are well known to those skilled in the art and may be performed, for example, using a method capable of high throughput operation, for example a chip-based method. New technology, called VLSIPS™, has enabled the production of extremely small chips that contain hundreds of thousands or more of different molecular probes. These biological chips or arrays have probes arranged in arrays, each probe assigned a specific location. Biological chips have been produced in which each location has a scale of, for example, ten microns. The chips can be used to determine whether target molecules interact with any of the probes on the chip. After exposing the array to target molecules under selected test conditions, scanning devices can examine each location in the array and determine whether a target molecule has interacted with the probe at that location.

Another method of identifying compounds with binding affinity for tenascin-C is the yeast two-hybrid system, where the polypeptides of the invention can be used to "capture" proteins that bind tenascin-C. The yeast two-hybrid system is described in Fields & Song, Nature 340:245-246 (1989).

In a further example, the agent is a compound which has ligand-binding capacity for tenascin-C.

For example, the agent may be a soluble fragment of an tenascin-C receptor (such as FPRL1). Alternatively, the agent may be a high affinity molecule that mimics an antibody (a so-called 'affibody') (for example, see US 5,831,012 and www.affibody.se). These ligands are small, simple proteins composed of a three-helix bundle based on the scaffold of one of the IgG-binding domains of Protein A (a surface protein from the bacterium *Staphylococcus aureus*). This scaffold has excellent features as an affinity ligand and can be designed to bind with high affinity to any given target protein.

The agent of the first aspect of the invention is an antibody or antigen-binding fragment thereof. The antigen-binding fragment may be selected from the group consisting of Fv fragments (*e.g.* single chain Fv and disulphide-bonded Fv), Fab-like fragments (*e.g.* Fab fragments, Fab' fragments and F(ab)₂ fragments), single variable domains (*e.g.* V_{H} and V_{L} domains) and domain antibodies (dAbs, including single and dual formats [*i.e.* dAb-linker-dAb]).

The antibody may preferably bind specifically to the FBG domain that activates TLR4.

The advantages of using antibody fragments, rather than whole antibodies, are several-foid. The smaller size of the fragments may lead to improved pharmacological properties, such as better penetration of solid tissue. Moreover, antigen-binding fragments such as Fab, Fv, ScFv and dAb antibody fragments can be expressed in and secreted from *E coli*, thus allowing the facile production of large amounts of the said fragments.

Also included within the scope of the invention are modified versions of antibodies and an antigen-binding fragments thereof, *e.g*. modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi. et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

Suitable-monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications" J G R Hurrell (CRC Press, 1982).

Antibody fragments can be obtained using methods well known in the art (see, for example, Harlow & Lane, 1988, "Antibodies: A Laboratory Manual", Cold Spring Harbor Laboratory, New York). For example, antibody fragments according to the present invention can be prepared by proteolytic hydrolysis of the antibody or by expression in *E. coli* or mammalian cells (*e.g.* Chinese hamster ovary cell culture or other protein expression systems) of DNA encoding the fragment. Alternatively, antibody fragments can be obtained by pepsin or papain digestion of whole antibodies by conventional methods.

It will be appreciated by persons skilled in the art that for human therapy or diagnostics, humanised antibodies are preferably used. Humanised forms of non-human (*e.g.* murine) antibodies are genetically engineered chimaeric antibodies or antibody fragments having preferably minimal-portions derived from non-human antibodies. Humanised antibodies include antibodies in which complementary determining regions of a human antibody (recipient antibody) are replaced by residues from a complementary determining region of a non human species (donor antibody) such as mouse, rat of rabbit having the desired functionality. In some instances, Fv framework residues of the human antibody are replaced by corresponding non-human residues. Humanised antibodies may also comprise residues which are found neither in the recipient antibody nor in the imported complementarity determining region or framework sequences. In general, the humanised antibody will comprise substantially all of at least one, and typically two, variable domains, in which all or substantially all of the complementarity determining regions correspond to those of a non human antibody and all, or substantially all, of the framework regions correspond to those of a relevant human consensus sequence. Humanised antibodies optimally also include at least a portion of an antibody constant region, such as an Fc region, typically derived from a human antibody (see, for example, Jones et al., 1986. Nature 321:522-525; Riechmann et a/., 1988, Nature 332:323-329; Presta, 1992, Curr. Op. Struct. Biol. 2:593-596).

Methods for humanising non-human antibodies are well known in the art. Generally, the humanised antibody has one or more amino acid residues introduced into it from a source which is non-human. These non-human amino acid residues, often referred to as imported residues, are typically taken from an imported variable domain. Humanisation can be essentially performed as described (see, for example, Jones et al., 1988, Nature 321:522-525; Reichmann et al., 1988. Nature 332:323-327; Verhoeyen et al., 1988, Science 239:1534-1536I; US 4,816,567) by substituting human complementarity determining regions with corresponding rodent complementarity determining regions. Accordingly, such humanised antibodies are chimaeric antibodies, wherein substantially less than an intact human variable domain has been substituted by the corresponding sequence from a non-human species. In practice, humanised antibodies may be typically human antibodies in which some complementarity determining region residues and possibly some framework residues are substituted by residues from analogous sites in rodent antibodies.

Human antibodies can also be identified using various techniques known in the art, including phage display libraries (see, for example, Hoogenboom & Winter, 1991, J. Mol. Biol. 227:381; Marks et al., 1991, J. Mol. Biol. 222:581; Cole et al., 1985, In: Monoclonal antibodies and Cancer Therapy, Alan R. Liss, pp. 77; Boerner et al., 1991. J. Immunol. 147:86-95).

Once suitable antibodies are obtained, they may be tested for activity, for example by ELISA.

The agent of the first aspect of the invention may be an antibody or antigen-binding fragment thereof has specificity for Toll Like Receptor 4 (TLR4), co-receptors of Toll Like Receptor 4 (in binding tenascin-4, tenascin-C or a domain thereof of any of these.

Co-receptors to primary receptors, such as TLR4, assist with binding of a signalling molecule to the primary receptor in order to facilitate ligand recognition and binding and initiate/maintain the biological process resulting from receptor binding.

The agent of the first aspect of the invention may be an antibody or antigen-binding fragment thereof has specificity for the FBG domain of tenascin-C.

Also described herein is a method of identifying an agent that modulates the activity of tenascin-C comprising the steps of:
(i) providing one or more a candidate agents;
(ii) contacting one or more cells with tenascin-C and the one or more candidate agents;
(iii) contacting one or more cells with tenascin-C and no candidate agent;
(iv) determining whether said candidate agent modulates the effect of tenascin-C on the one or more cells in step (ii) in comparison to the cell(s) of control step (iii).

Methods of determining whether the candidate agent modulate the effect of tenascin-C can be carried out using the methods of the examples.

The method may result in the activity of tenascin-C being upregulated.

The method may result in the activity of tenascin-C being downregulated.

The method may include the cells of steps (ii) and (iii) (described above) expressing Toll-like receptor 4 (TLR4).

The method may have the one or more cells selected from the group consisting of inflammatory cells, fibroblasts, fibroblast like cells (including RA synovial fibroblasts, also known as synoviocytes), mouse embryonic fibroblasts, human embryonic kidney cells.

The inflammatory cells may be selected from the group consisting of macrophages, dendritic cells, monocytes, lymphocytes, monocyte like cells and macrophage like cells.

Also described herein is a method identification of an agent that modulates a chronic inflammatory response by conducting the method of identifying an agent that modulates the activity of tenascin-C.

In this method the chronic inflammation may be associated with any condition associated with inappropriate inflammation. Such conditions include, but are not limited to, rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

Of particular, but non-exclusive interest, the chronic inflammation is associated with rheumatoid arthritis (RA).

Also described herein is an agent identified according to the method of the second and third aspects of the invention. Such an agent may modulate a chronic inflammatory response.

The agent may down-regulate the chronic inflammatory response.

The agent may up-regulate the chronic inflammatory response.

The agent may be selected from the group consisting of short interfering RNA (SiRNA) molecules, short hairpin RNA molecules (shRNA), antisense oligonucleotides, compounds with binding affinity for tenascin-C, antibodies (polyclonal or monoclonal) and antigen-binding fragments thereof, small inhibitor compounds, polypeptides and proteins.

In the first aspect of the invention the chronic inflammation may be associated with any condition associated with inappropriate inflammation. Such conditions include, but are not limited to, rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

In a second aspect of the invention there is provided a composition comprising an agent as defined in the first aspect of the invention and a pharmaceutically acceptable carrier, excipient and/or diluent.

It will be appreciated by persons skilled in the art that such an effective amount of the agent or formulation thereof may be delivered as a single bolus dose (i.e. acute administration) or, more preferably, as a series of doses over time (i.e. chronic administration).

The agents of the invention can be formulated at various concentrations, depending on the efficacy/toxicity of the compound being used and the indication for which it is being used. Preferably, the formulation comprises the agent of the invention at a concentration of between 0.1 µM and 1 mM, more preferably between 1 µM and 100 µM, between 5 µM and 50 µM, between 10 µM and 50 µM, between 20 µM and 40 µM and most preferably about 30 µM. For *in vitro* applications, formulations may comprise a lower concentration of a compound of the invention, for example between 0.0025 µM and 1 µM.

It will be appreciated by persons skilled in the art that the agents of the invention will generally be administered in admixture with a suitable pharmaceutical excipient diluent or carrier selected with regard to the intended route of administration and standard pharmaceutical practice (for example, see Remington: The Science and Practice of Pharmacy, 19th edition, 1995, Ed. Alfonso Gennaro, Mack Publishing Company, Pennsylvania; USA).

For example, the agents of the invention can be administered orally, buccally or sublingually in the form of tablets, capsules, ovules, elixirs, solutions or suspensions, which may contain flavouring or colouring agents, for immediate-, delayed- or controlled-release applications. The agents of invention may also be administered via intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch; cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The agents of the invention can also be administered parenterally, for example, intravenously, intra-articularly, intra-arterially, intraperitoneally, intra-thecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

For oral and parenteral administration to human patients, the daily dosage level of the agents of the invention will usually be from 1 to 1000 mg per adult (*i.e.* from about 0.015 to 15 mg/kg), administered in single or divided doses.

The agents of the invention can also be administered intranasally or by inhalation and are conveniently delivered in the form of a dry powder inhaler or an aerosol spray presentation from a pressurised container, pump, spray or nebuliser with the use of a suitable propellant, *e.g*. dichlorodifluoromethane, trichlorofluoro-methane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, *e.g*. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or 'puff' contains at least 1 mg of a compound of the invention for delivery to the patient. It will be appreciated that the overall daily dose with an aerosol will vary from patient to patient, and may be administered in a single dose or, more usually, in divided doses throughout the day.

Alternatively, the agents of the invention can be administered in the form of a suppository or pessary, or they may be applied topically in the form of a lotion, solution, cream, ointment or dusting powder. The compounds of the invention may also be transdermally administered, for example, by the use of a skin patch. They may also be administered by the ocular route.

For ophthalmic use, the agents of the invention can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the agents of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Where the agent is a polypeptide, it may be preferable to use a sustained-release drug delivery system, such as a microspheres. These are designed specifically to reduce the frequency of injections. An example of such a system is Nutropin Depot which encapsulates recombinant human growth hormone (rhGH) in biodegradable microspheres that, once injected, release rhGH slowly over a sustained period.

Alternatively, polypeptide agents of the present invention can be administered by a surgically implanted device that releases the drug directly to the required site.

Electroporation therapy (EPT) systems can also be employed for the administration of proteins and polypeptides. A device which delivers a pulsed electric field to cells increases the permeability of the cell membranes to the drug, resulting in a significant enhancement of intracellular drug delivery.

Proteins and polypeptides can also be delivered by electroincorporation (EI). El occurs when small particles of up to 30 microns in diameter on the surface of the skin experience electrical pulses identical or similar to those used in electroporation. In El, these particles are driven through the stratum corneum and into deeper layers of the skin. The particles can be loaded or coated with drugs or genes or can simply act as "bullets" that generate pores in the skin through which the drugs can enter.

An alternative method of protein and polypeptide delivery is the thermo-sensitive ReGel injectable. Below body temperature, ReGel is an injectable liquid while at body temperature it immediately forms a gel reservoir that slowly erodes and dissolves into known, safe, biodegradable polymers. The active drug is delivered over time as the biopolymers dissolve.

Protein and polypeptide pharmaceuticals can also be delivered orally. One such system employs a natural process for oral uptake of vitamin B12 in the body to co-deliver proteins and polypeptides. By riding the vitamin B12 uptake system, the protein or polypeptide can move through the intestinal wall. Complexes are produced between vitamin B12 analogues and the drug that retain both significant affinity for intrinsic factor (IF) in the vitamin B12 portion of the complex and significant bioactivity of the drug portion of the complex.

Methods for administering oligonucleotide or polynucleotide agents of the invention are also well know in the art (see Dass, 2002, J Pharm Pharmacol. 54(1):3-27; Dass, 2001, Drug Deliv. 8(4):191-213; Lebedeva et al., 2000, Eur J Pharm Blopharm. 50(1):101-19; Pierce et al., 2005, Mini Rev Med Chem. 5(1):41-55; Lysik & Wu-Pong, 2003, J Pharm Sci. 2003 2(8):1-559-73; Dass, 2004, Biotechnol Appl Biochem. 40(Pt 2):113-22; Medina, 2004, Curr Pharm Des. 10(24):2981-9.

The composition of the second aspect of the invention may further comprise at least one other agent.

Such a further agent may be an anti-inflammatory agent which includes but is not limited to non-steroidal anti-inflammatory agent (NSAID), a disease modifying anti-rheumatic drug (DMARD), a statin (including HMG-CoA reductase inhibitors such as simvastatin), a biological agent (biologicals), a steroid, an immunosuppressive agent, a salicylate and/or a microbicidal agent. Non-steroidal anti-inflammatory agents include anti-metabolite agents (such as methotrexate) and anti-inflammatory gold agents (including gold sodium thiomalate, aurothiomalate or gold salts, such as auranofin). Biologicals include anti-TNF agents (including adalimumab, etanercept, infliximab, anti-IL-1 reagents, anti-IL-6 reagents, anti-B cell reagents (retoximab), anti-T cell reagents (anti-CD4 antibodies), anti-IL-15 reagents, anti-CLTA4 reagents, anti-RAGE reagents), antibodies, soluble receptors, receptor binding proteins, cytokine binding proteins, mutant proteins with altered or attenuated functions, RNAi, polynucleotide aptemers, antisense oligonucleotides or omega 3 fatty acids. Steroids (also know as corticosteroids) include cortisone, prednisolone or dexamethasone. Immunosuppressive agents include cylcosporin, FK506, rapamycin, mycophenolic acid. Salicylates include aspirin, sodium salicylate, choline salicylate and magnesium salicylate. Microbicidal agents include quinine and chloroquine. For example, the agent may be administered in combination with one or more of an NSAID, DMARD, or immunosuppressant

Also described herein is an agent or composition as defined herein for use as a medicament.

In a third aspect of the invention there is provided an agent or composition as defined in the first, or second aspects of the invention for use in the treatment of a chronic inflammatory condition, wherein the chronic inflammatory response is associated with rheumatoid arthritis (RA), inflammatory bowel diseases, atherosclerosis and/or psoriasis.

In a fourth aspect of the invention there is provided the use of an agent or composition as defined in as defined in the first, or second aspects of the invention in the manufacture of a medicament for the treatment of a chronic inflammatory condition, wherein the chronic inflammatory response is associated with rheumatoid arthritis (RA), inflammatory bowel diseases, atherosclerosis and/or psoriasis.

Also described herein is a method of treating a chronic inflammatory condition comprising administering to a subject an effective amount of an agent or composition as defined herein.

The agent, composition, use or method as defined herein may relate to treatment of a chronic inflammatory condition wherein the condition is associated with any condition associated with inappropriate inflammation. Such conditions include, but are not limited to, rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

Also described herein is a kit of parts for performing the methods described herein comprising:
(i) one or more cells
(ii) a control sample of one or more cells
(iii) a sample of tenascin-C
(iv) instructions for their use

The kit may optionally comprise:
(v) a candidate agent.

The kit may further optionally comprise
(vi) means of determining the effect of a candidate agent on either tenascin-C activity or chronic inflammation.

In a fifth aspect of the invention there is provided a kit of parts comprising:
(i) an agent or composition as defined in the first or second aspects of the invention
(ii) administration means
(iii) instructions for their use

The kit of the fifth aspect of the invention may further optionally comprise
(iv) at least one other agent.

### Definitions

By "inflammation" we include the meaning of local accumulation of fluid, plasma proteins, and white blood cells that is initiated by tissue injury, infection or a local immune response.

By "acute inflammation" we include the meaning of the initial stages (initiation) of inflammation and the short-term transient inflammatory response immediately after injury, infection or local immune response. Typically, acute inflammation is rapidly resolved, lasting from a matter of minutes to no longer that a few days.

By "chronic inflammation" we include the meaning of persistent and/or non-resolved inflammation. It is often associated with inappropriate destruction of healthy tissue. This may be progressive and last over a period of weeks or longer. Chronic inflammation is typically associated with persistent infection or disease including, but not limited to, automimmune conditions.

By "chronic joint inflammation" we include the meaning of persistent inflammation that is progressive and unremitting over a period of weeks to months, resulting in distortion of the affected joint and radiographic evidence of cartilage and bone destruction as observed in human disease (Kelly, Harris, Ruddy and Sledge, Textbook of Rheumatology 4th Edition).

In experimental murine models, chronic joint inflammation is characterised by inflammation that does not subside and causes inappropriate tissue destruction, even over a relatively short period of time. This is characterized (and can be identified) histologically by the prolonged presence of inflammatory cells in the synovium and joint space, chondrocyte death, and cartilage and bone erosion.

By an "agent" we include all chemical entities, for example oligonucleotides, polynucleotide, polypeptides, peptidomimetics and small compounds.

By "fragment" we mean at least 10 nucleotides, for example at least 15, 16, 17, 18, 19, 20, 21, 22, 23, 24 or 25 nucleotide.

By "variant" we mean that the nucleotide sequence shares at least 90% sequence identity with the full length sequence of interest, for example at least 50%, 55%, 60%, 65%, 70%, 75%, 80%, 85%, 90%, 95%, 96%, 97%, 98% or 99% sequence identity.

The percent sequence identity between two polynucleotides may be determined using suitable computer programs, for example the GAP program of the University of Wisconsin Genetic Computing Group and it will be appreciated that percent identity is calculated in relation to polynucleotides whose sequences have been aligned optimally.

The alignment may alternatively be carried out using the Clustal W program (as described in Thompson et al., 1994, Nuc. Acid Res. 22:4673-4680).

The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05.
Scoring matrix: BLOSUM.

Alternatively, the BESTFIT program may be used to determine local sequence alignments.

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

By "antigen-binding fragment" we mean a functional fragment of an antibody that is capable of binding to tenascin-C.

The term "subject" means all animals including humans. Examples of subjects include humans, cows, dogs, cats, goats, sheep, and pigs. The term "patient" means a subject having a disorder in need of treatment.

As used herein, 'pharmaceutical formulation' means a therapeutically effective formulation according to the invention.

A 'therapeutical effective amount', or 'effective amount', or 'therapeutical effective', as used herein, refers to that amount which provides a therapeutic effect for a given condition and administration regimen. This is a predetermined quantity of active material calculated to produce a desired therapeutic effect in association with the required additive and diluent, *i.e*. a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, etc., as is well known in the art.

Examples embodying an aspect of the invention will now be described with reference to the following figures in which:

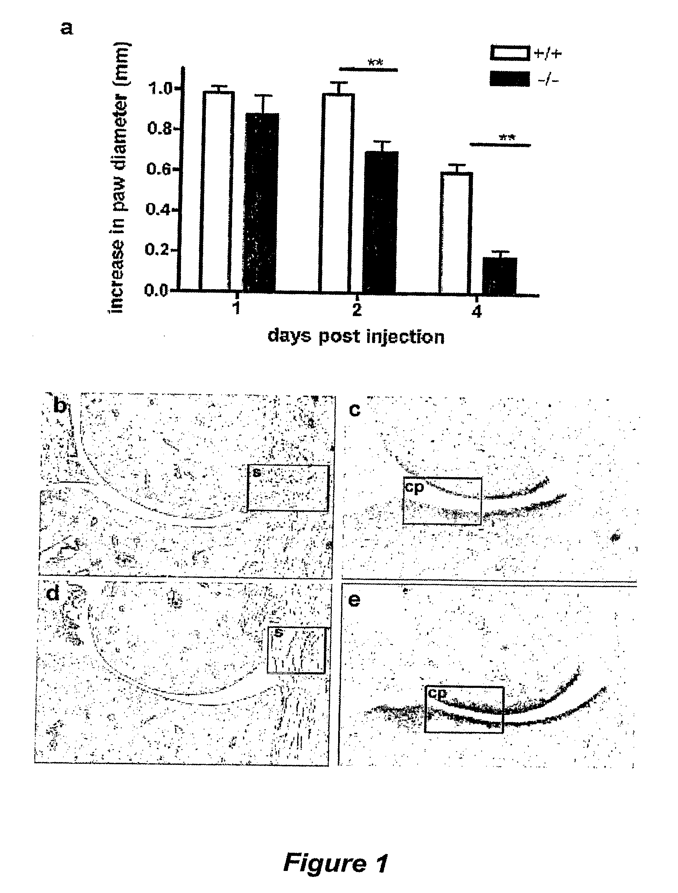

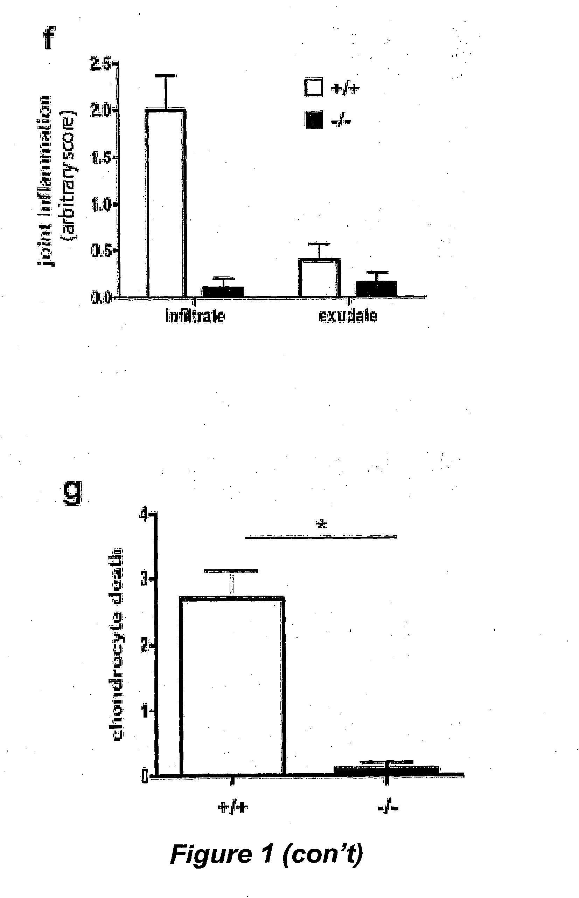

**Figure 1****. Accelerated resolution of acute inflammation in tenascin-C deficient mice.**
   (a) Paw swelling in wild type (+/+)(white bars) and tenascin-C null (-/-)(black bars) mice over time after injection of zymosan. Data are shown as the mean increase in paw diameter compared to paw diameter before injection +/-SEM (n = 24 mice per genotype). **= p<0.01. (b-e) Representative sections of the ankle joint from wild type (b, c) and tenascin-C null (d, e) mice 4 days after zymosan injection, stained with hemotoxylin and eosin (b, d) and safranin-O (c, e). Boxes highlight the joint synovium (s) and cartilage proteoglycan (cp). Magnification x10. Quantification of joint inflammation (f) and chondrocyte death (g) in knee joints 4 days after injection with zymosan from wild type mice (white bars) and tenascin-C null mice (black bars). Data are expressed as the mean (+/- SD) (n = 24 mice per genotype). *= p <0.05.

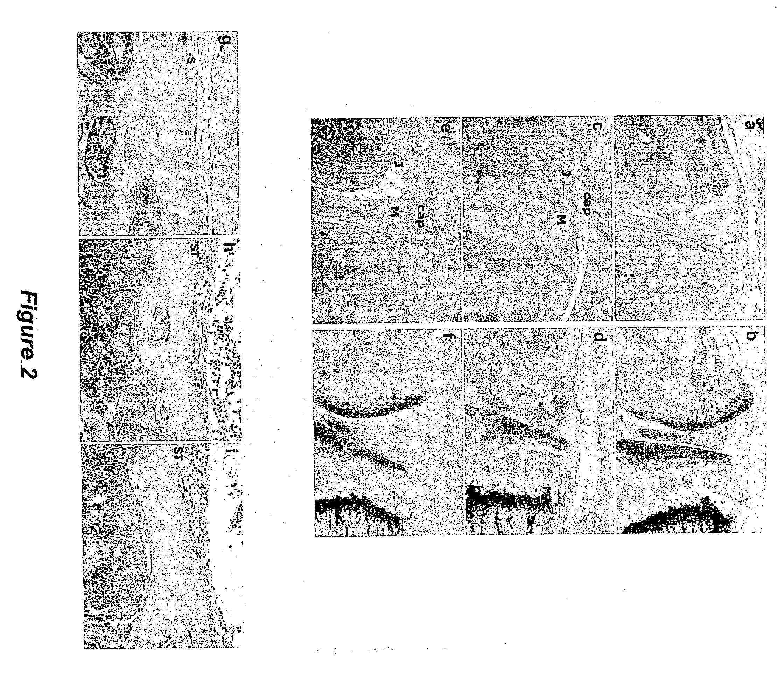

**Figure 2****. Synovial inflammation is induced in tenascin-C deficient mice upon injection of antigen.**
   (a-b, g) Representative sections of the knee joint of sham injected wild type mice. (c-f, h-i) Representative sections of the knee joint of wild type (c, d, h) or tenascin-C null (e, f, i) mice 24 hours after intra-articular injection of mBSA. Inflammatory cell infiltration in the capsule, meniscus and the joint space of both wild type and tenascin-C null mice is highlighted by (cap), (M) and (J) respectively. (S) highlights the healthy synovium of sham injected mice that is no more than 1-3 cells thick along the entire bone surface and (ST) highlights the synovia of wild type and tenascin-C null mice which are both significantly thickened. Sections are stained with hemotoxylin and eosin (a, c, e, g, h, i) and safranin-O (b, d, f). Magnification x10 (a-f) or x40 (g-i). (n = 5 mice per genotype).

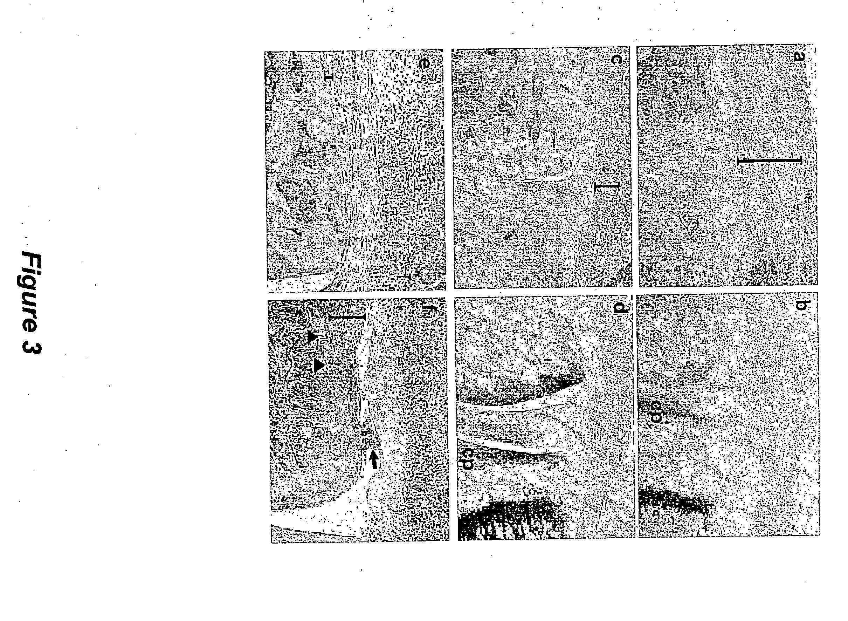

**Figure 3****. Synovial inflammation subsides rapidly in tenascin-C deficient mice.**
   Representative sections of the knee joint of wild type (a, b, f) or tenascin-C null (c, d, e) mice 3 days after intra-articular injection of mBSA. (a, c) The line highlights increased inflammation of the capsule in wild type mice compared to tenascin-C null mice. (b, d) (cp) highlights increased cartilage proteoglycan loss in wild type mice compared to tenascin-C null mice. (e, f) Significant synovial hyperplasia (line), cell and fibrin deposits in the joint space (arrow) and pannus invasion (arrow heads) are observed in wild type mice compared to tenascin-C null mice. Sections are stained with hemotoxylin and eosin (a, c, e, f) and safranin-O (b, d) Magnification x10 (a-d) or x20 (e-f). (n = 5 mice per genotype).

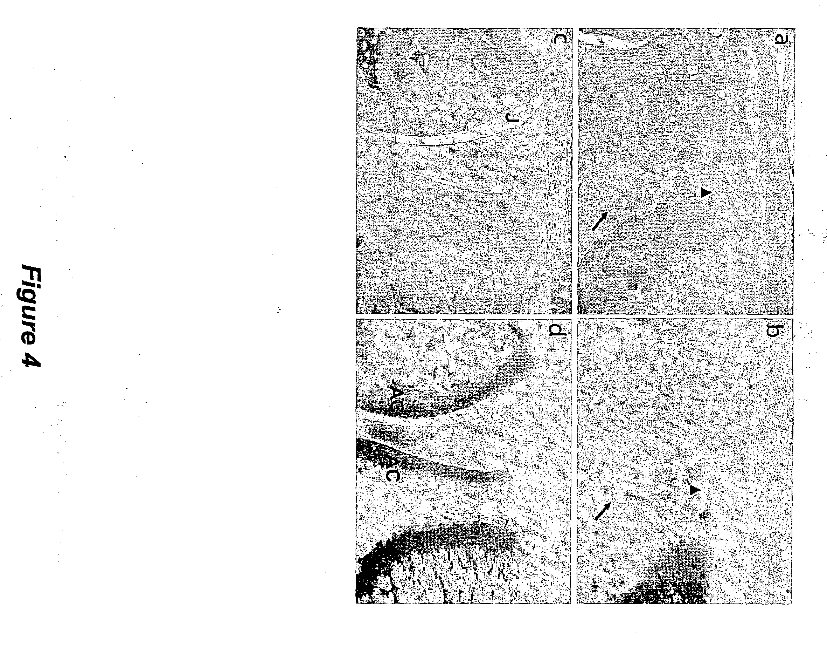

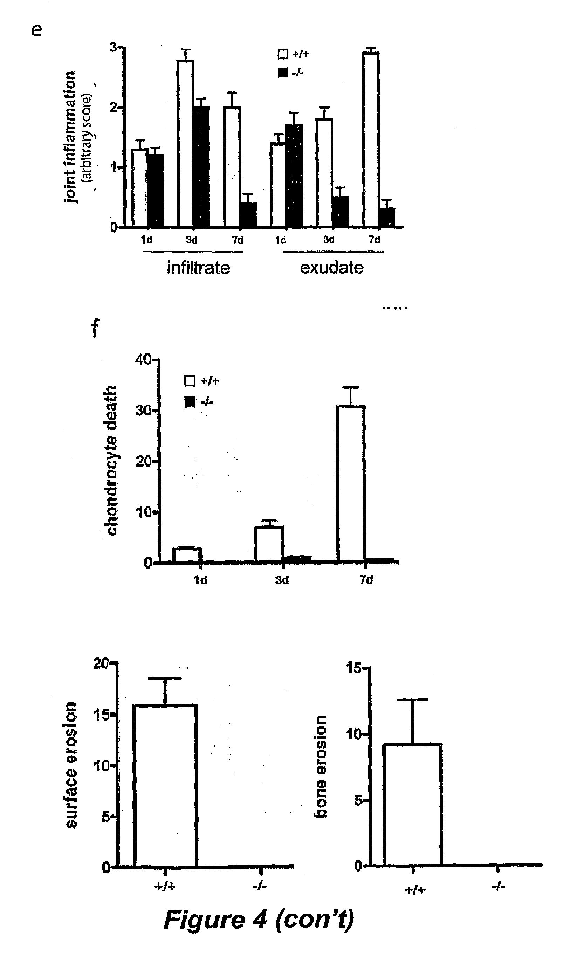

**Figure 4****. tenascin-C deficient mice are protected from tissue destruction during antigen induced arthritis.**
   (a-b) Representative sections of the knee joint of wild type mice 7 days after intra-articular injection of mBSA, stained with hemotoxylin and eosin (a) and safranin-O (b). Magnification x10. (n = 24 mice per genotype). Arrowhead highlights area of bone erosion. Arrow highlights pannus invasion into articular cartilage. (c-d) Representative sections of the knee joint of tenascin-C null type mice 7 days after intra-articular injection of mBSA, stained with hemotoxylin and eosin (c) and safranin-O (d). Magnification x10. (n = 24 mice per genotype). J highlights the joint space and AC the intact articular cartilage. (e) Histological score of knee joint inflammation 24 hours, 3 days and 7 days after injection with mBSA from wild type mice (white bars) and tenascin-C null mice (black bars). Data represent the mean +/- SD (n = 5 per genotype (24h, 3d) or 24 per genotype (7d)). (f) Quantification of chondrocyte death, cartilage surface erosion and bone erosion after injection with mBSA in knee joints from wild type mice (white bars) and tenascin-C null mice (black bars). Chondrocyte death is shown at 24 hours, 3 days and 7 days, and cartilage surface erosion and bone erosion at 7d. Data represent the mean +/- SD (n = 5 per genotype (24h, 3d) or 24 per genotype (7d)).

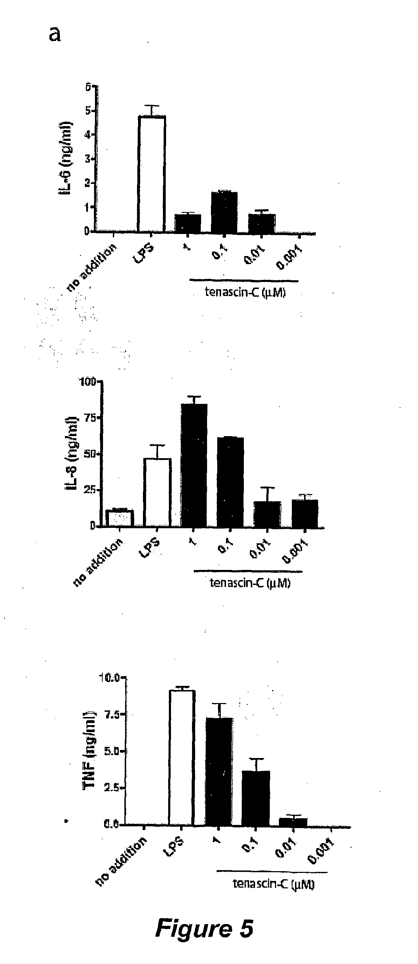

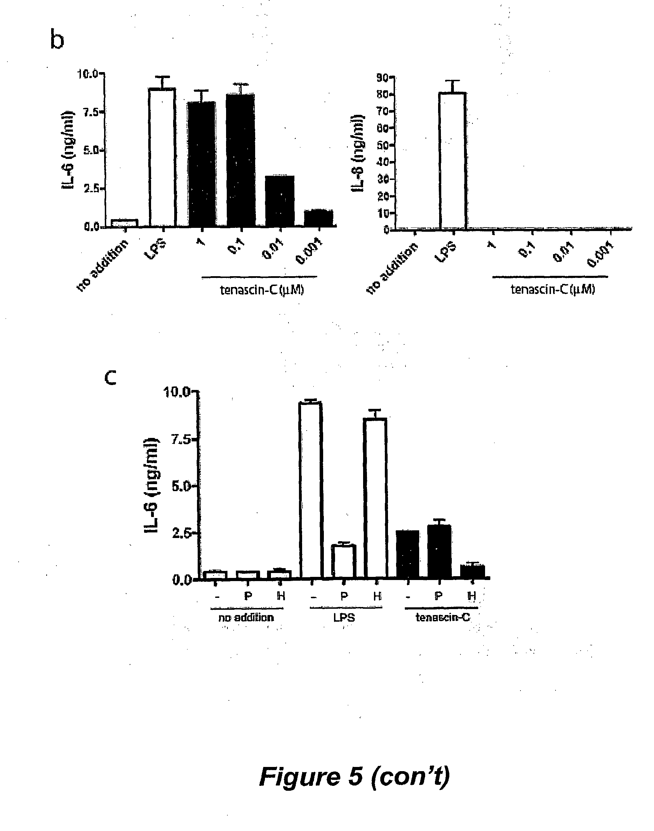

**Figure 5****. tenascin-C induces TNF-α, IL-6 and IL-8 synthesis in primary human macrophages and RA synovial fibroblasts.**
   (a-b) Primary human macrophages (a) and RA synovial fibroblasts (b) were unstimulated (no addition) or stimulated with LPS (1 ng/ml (a) or 10 ng/ml (b)) or recombinant tenascin-C (1.0 µM - 1.0 nM) for 24h. Data shown are the mean of triplicate values (+/-SD) from one of three representative experiments. (c) Primary human macrophages were unstimulated (no addition) or stimulated with LPS (1 ng/ml) or recombinant tenascin-C (1.0 µM) for 24h. (-) indicates cells were pre-incubated with medium alone. (P) Cells were pre-incubated with 25 µg/ml polymyxin B for 30 min before stimulation. (H) Cells were incubated with medium with no addition or containing LPS or tenascin-C that was boiled for 15 minutes before addition to cells. Data shown are the mean of triplicate values (+/- SD) from one of three representative experiments.

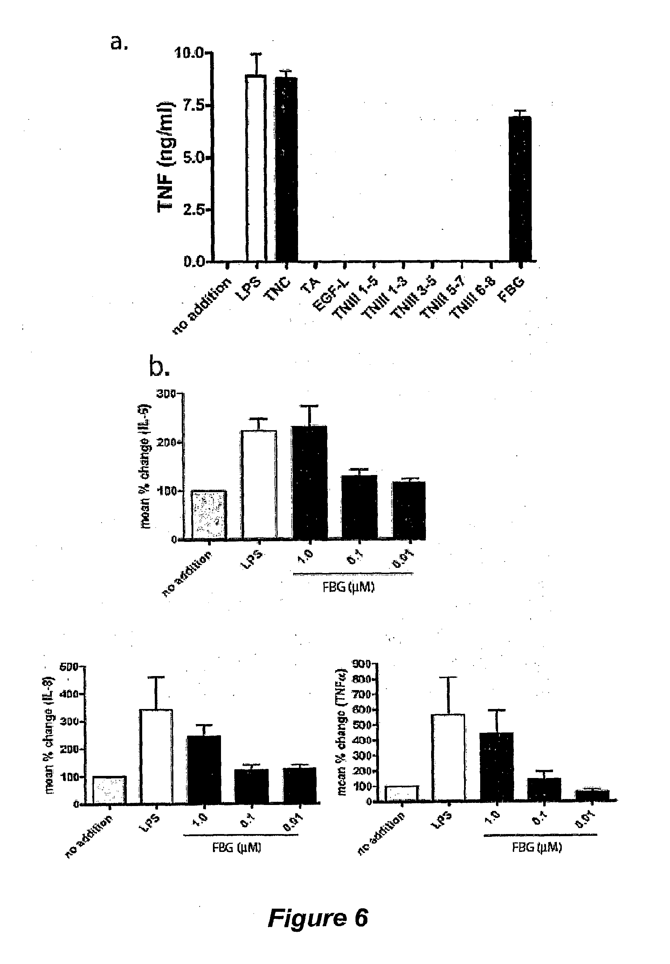

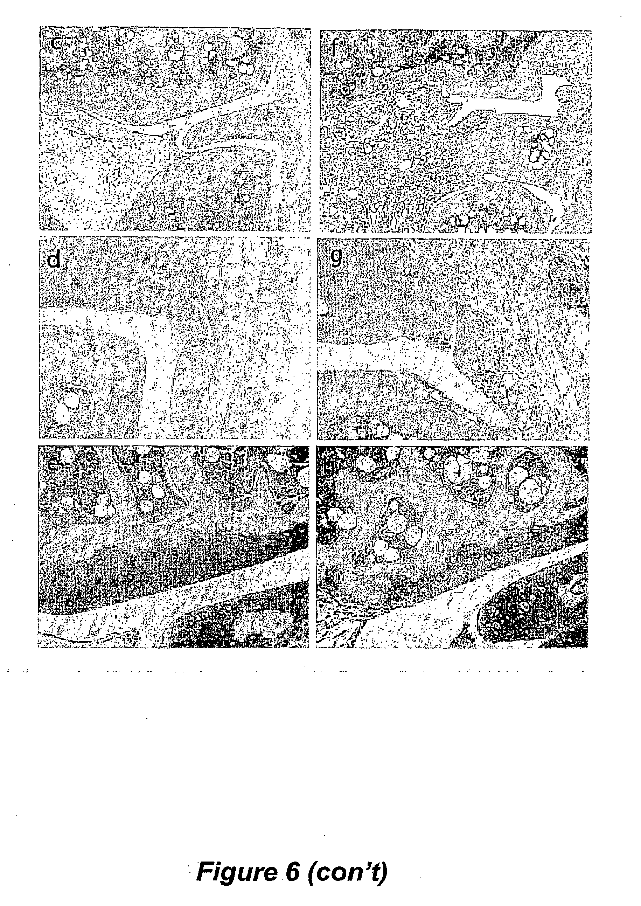

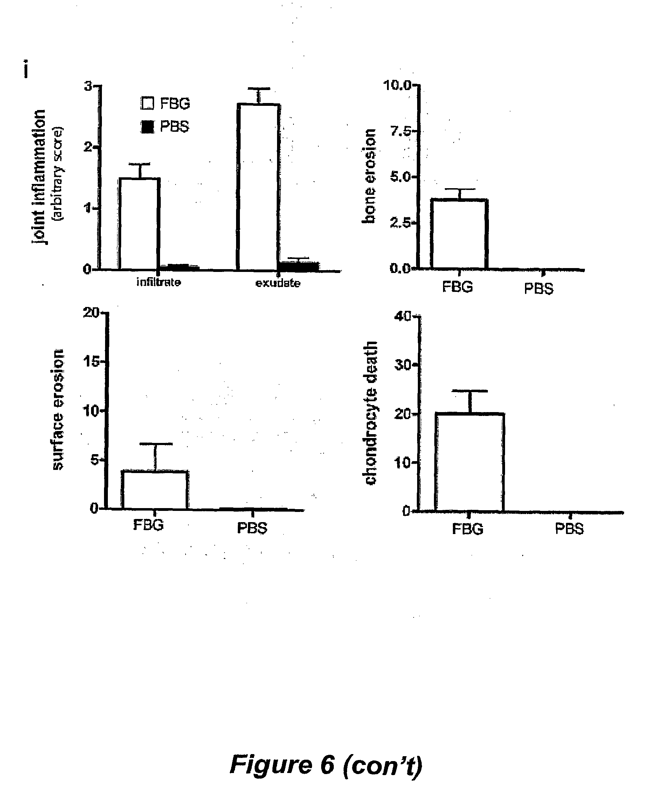

**Figure 6****. The FBG domain of tenascin-C mediates stimulation of cytokine synthesis *in vivo* and *in vitro.***
   (a) Primary human macrophages were unstimulated (no addition) or stimulated with LPS (1 ng/ml), recombinant tenascin-C (TNC) or 1.0 µM tenascin-C domains (TA, EGF-L, TNIII1-5, TNIII1-3, TNIII3-5, TNIII5-7, TNIII6-8 and FBG) for 24h. Data shown are the mean of triplicate values (+/- SD) from one of three representative experiments. (b) RA synovial membrane cells were unstimulated (no addition) or stimulated with LPS (10 ng/ml) or recombinant FBG (1.0 - 0.01 µM) for 24h. Data shown are the mean % change in cytokine levels compared to unstimulated cells (+/- SEM) from five different patients. (c-h) Representative sections of the knee joint of wild type mice 3 days after intra-articular injection of PBS (c-e) or 1 µg FBG (f-h). Sections are stained with hemotoxylin and eosin (c,d,f,g) or Safranin-O (e, h). Magnification x10 (c, f) or x25 (d,e,g,h) (n = 5 mice per genotype). (i) Quantification of joint inflammation, bone erosion, cartilage surface erosion and chondrocyte death in the knee joints of wild type mice 3 days after intra-articular injection of PBS (black bars) or 1 µg FBG (white bars). Data represent the mean +/- SD (n = 5 per genotype).

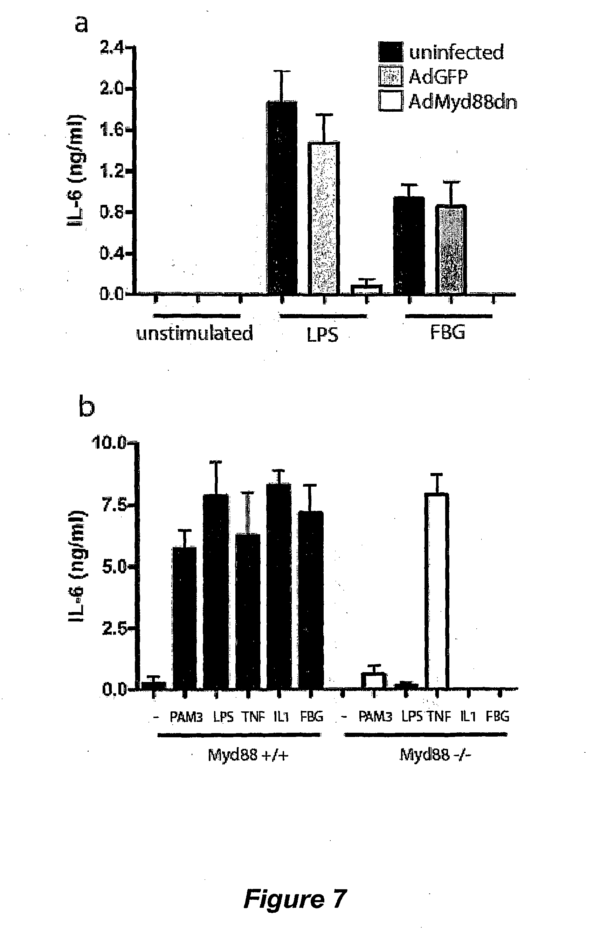

**Figure 7****. FBG mediated cytokine synthesis is MyD88 dependent.**
   (a) Human RA synovial fibroblasts were either uninfected, infected with adenovirus expressing GFP alone (AdGFP) or infected with adenovirus expressing dominant negative MyD88 (AdMyD88dn). Cells were unstimulated, stimulated with LPS (10 ng/ml) or stimulated with FBG (1µM) for 24h. Data shown are the mean of three independent experiments (+/- SEM). (b) Mouse embryonic fibroblasts isolated from wild type (+/+) or MyD88 deficient (-/-) mice were unstimulated (-) or stimulated with PAM3 (100 ng/ml), LPS (100 ng/ml), TNFα (100 ng/ml), IL-1 (5 ng/ml) and FBG (1 µM) for 24h. Data shown are the mean of three independent experiments (+/-SEM).

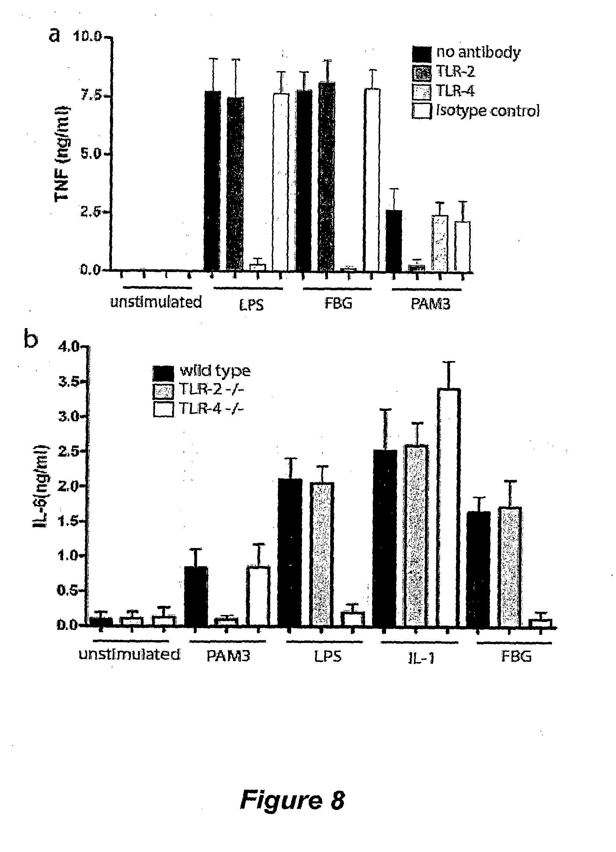

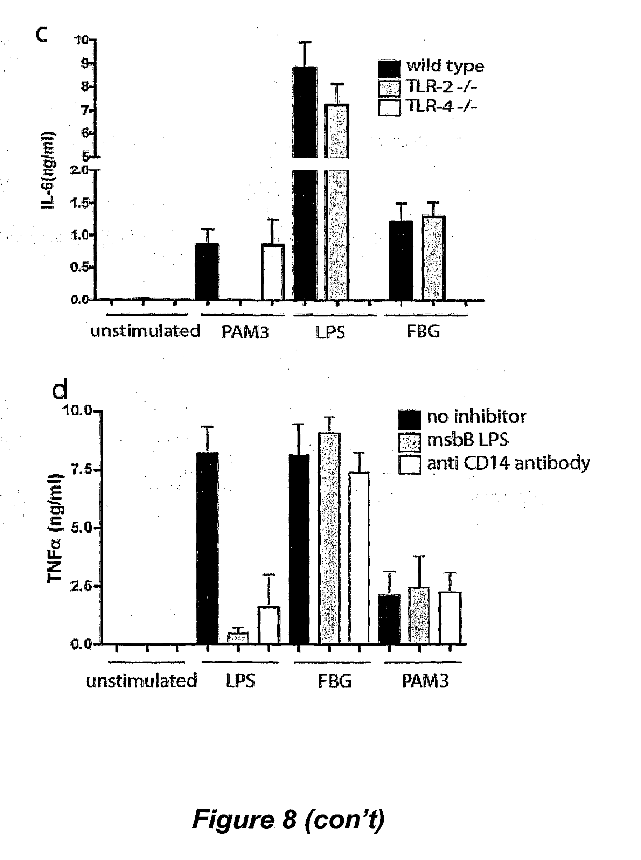

**Figure 8****. FBG mediated cytokine synthesis is TLR4 dependent but does not require CD14 or MD-2.**
   (a) Primary human macrophages were pre-incubated with medium alone or medium containing function blocking antibodies to TLR2 (10 µg/ml), TLR4 (25 µg/ml) or isotype control antibodies (25 µg/ml) for 30 min before stimulation. Cells were unstimulated, or stimulated with LPS (1 ng/ml), FBG (1 µM) or PAM3 (10 ng/ml) for 24h. Data shown are the mean of three independent experiments (+/-SEM). (b) Mouse embryonic fibroblasts isolated from wild type, TLR2 (TLR2 -/-) or TLR4 (TLR4 --/-) deficient mice were unstimulated or stimulated with PAM3 (100 ng/ml), LPS (100 ng/ml), IL-1 (5 ng/ml) and FBG (1 µM) for 24h. Data shown are the mean of three independent experiments (+/-SEM). (c) Bone marrow derived macrophages isolated from wild type, TLR2 (TLR2 -/-) or TLR4 (TLR4 -/-) deficient mice were unstimulated or stimulated with PAM3 (100 ng/ml), LPS (100 ng/ml) or FBG (1 µM) for 24h. Data shown are the mean of three independent experiments (+/-SEM). (d) Human macrophages were pre-incubated with no inhibitor, 1µg/ml msbB LPS or 10µg/ml anti-CD14 antibody for 30 min before stimulation with LPS (1 ng/ml), FBG (1 µM) or PAM3 (10 ng/ml) for 24h. Data shown are the mean of three independent experiments (+/-SEM).
**Figure 9****. Paw swelling over time after injection of zymosan.**
   Representative images of the paws of non-injected tenascin-C null mice (a, e) (diameter 1.6 mm), tenascin-C null mice 24h (d, f) (diameter 2.5 mm) and 4d (b, h) (diameter 1.7 mm) after zymosan injection and from wild type mice 4d after zymosan injection (c, g) (diameter 2.1 mm).
**Figure 10****. Synthesis of recombinant proteins.**
   (a) Domain structure of the tenascin-C monomer comprising different domains, including the assembly domain (TA), 14 and a half EGF-like repeats (EGF-L), 17 fibronectin type III-like repeats (TNIII) (8 constitutively expressed (1-8) and 9 that can be alternatively spliced, and a fibrinogen-like globe (FBG). (b) The regions covered by the recombinant proteins that were synthesized, the corresponding amino acid residues and the molecular weight of each protein.

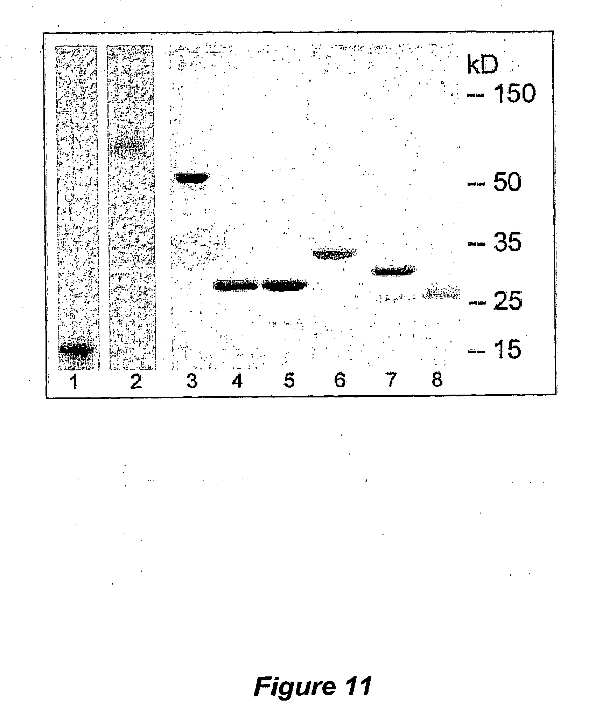

**Figure 11****. Analysis of protein purity.**
   Silver stained gel showing 1 µg of each recombinant protein analysed by SDS-PAGE under reducing conditions. Lanes: 1 (TA), 2 (EGF-L), 3 (TNIII1-5), 4 (TNIII5-7), 5 (TNIII6-8), 6 (TNIII1-3), 7 (TNIII3-6) and 8 (FBG).

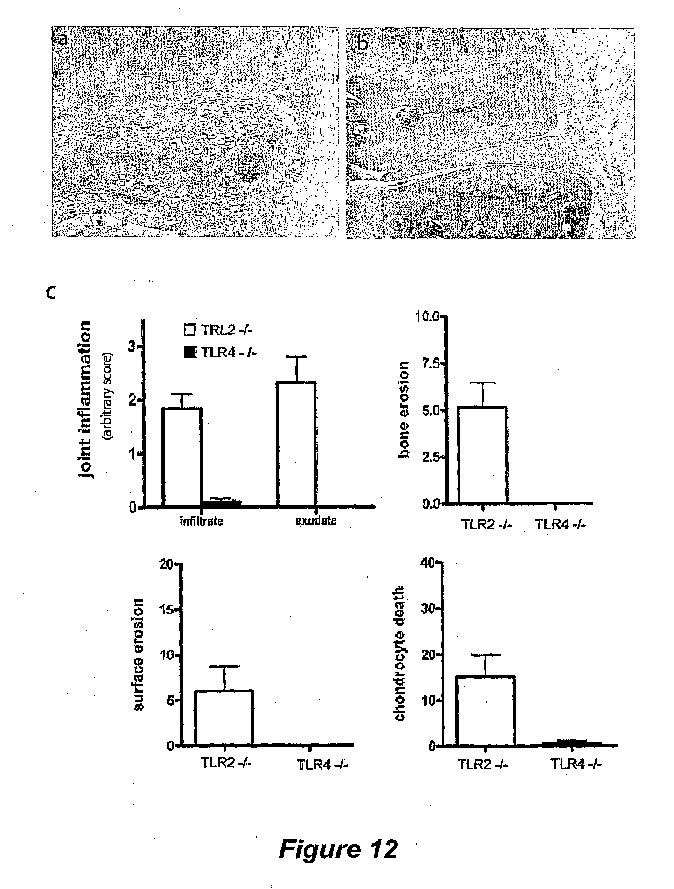

**Figure 12****. FBG-mediated joint inflammation in vivo requires expression of TLR4.**
   Representative sections of the knee joint of TLR2 (a) and TLR4 (b) null mice 3 days after intra-articular injection of 1 µg FBG. Sections are stained with hemotoxylin and eosin. Magnification x10 (n = 5 mice per genotype). (c) Quantification of joint inflammation, bone erosion, cartilage surface erosion and chondrocyte death in the knee joints of TLR2 (white bars) and TLR4 (black bars) null mice 3 days after intra-articular injection of 1 µg FBG. Data represent the mean +/- SD (n = 5 per genotype).
**Figure 13****. Amino acid sequence of human tenascin-C and its domains**
**Figure 14****. Nucleotide sequence of human tenascin-C**

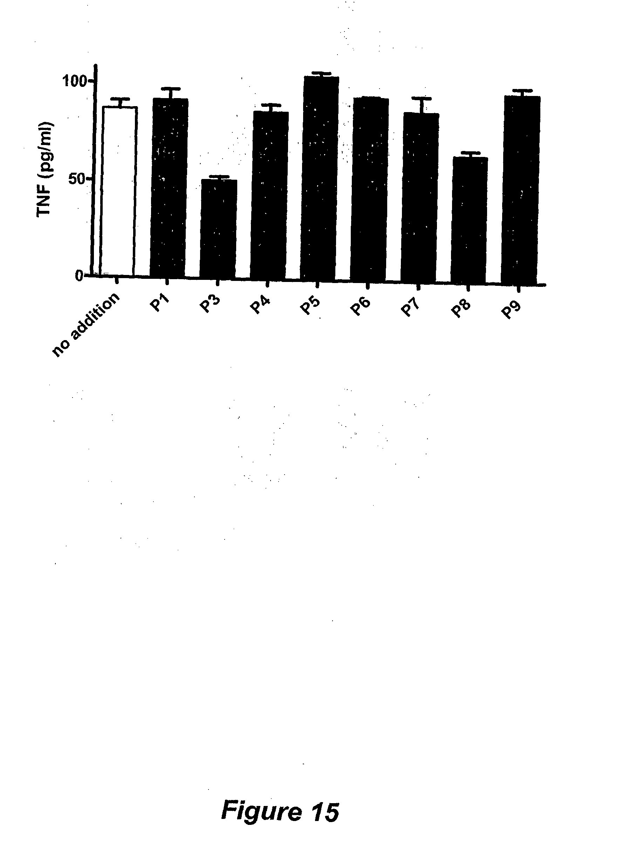

**Figure 15****. TNF synthesis in response to specific FBG peptides.**
   TNF synthesis by RA membrane cultures incubated for 24h with no addition or 100 µM of each FBG peptide (P1, P3-P9).

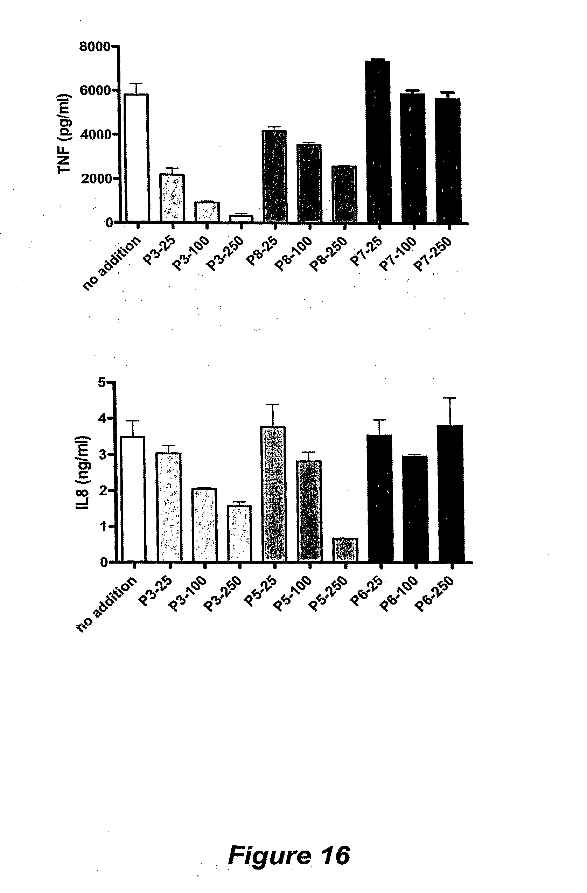

**Figure 16****. TNF and IL8 synthesis in response to varying concentrations of specific FBG peptides.**
   TNF & IL8 synthesis by RA membrane cultures incubated for 24h with no addition or 25, 100 or 250 µM of FBG peptide.

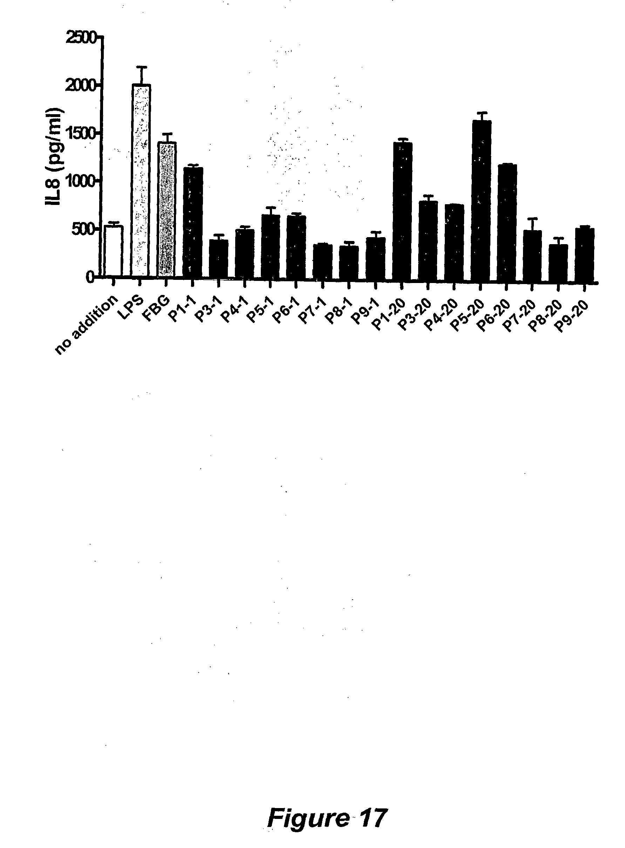

**Figure 17****. IL8 synthesis in response to LPS, whole FBG domain or specific FBG peptides.**
   IL8 synthesis by macrophages after 24h incubation with no addition, 1 ng/ml LPS, 1 µM whole FBG domain (FBG) or 1 or 20 µM of FBG peptides (P1, P3-P9).

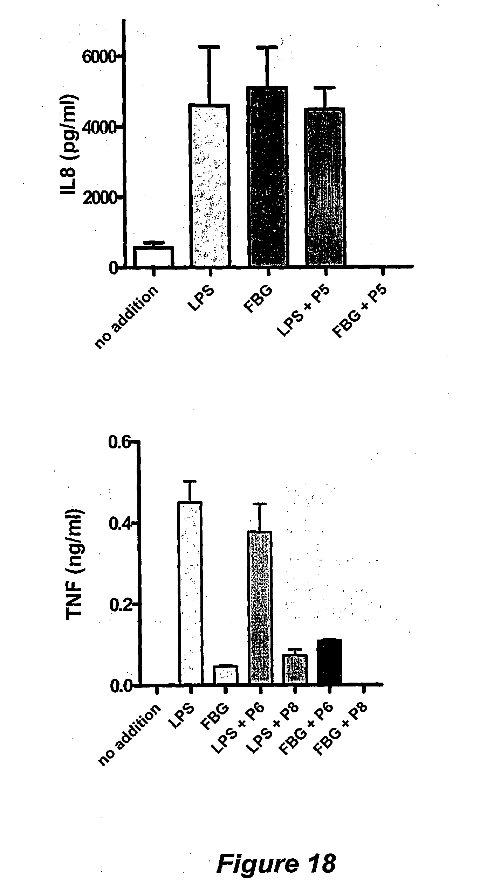

**Figure 18****. IL8 and TNF synthesis in response to LPS and FBG following pre-incubation with FBG peptides.**
   TNF and IL8 synthesis by macrophages after 24h incubation with no addition, 1 ng/ml LPS or 1 µM whole FBG domain (FBG), either with or without pre-incubation with 20 µM of FBG peptides.

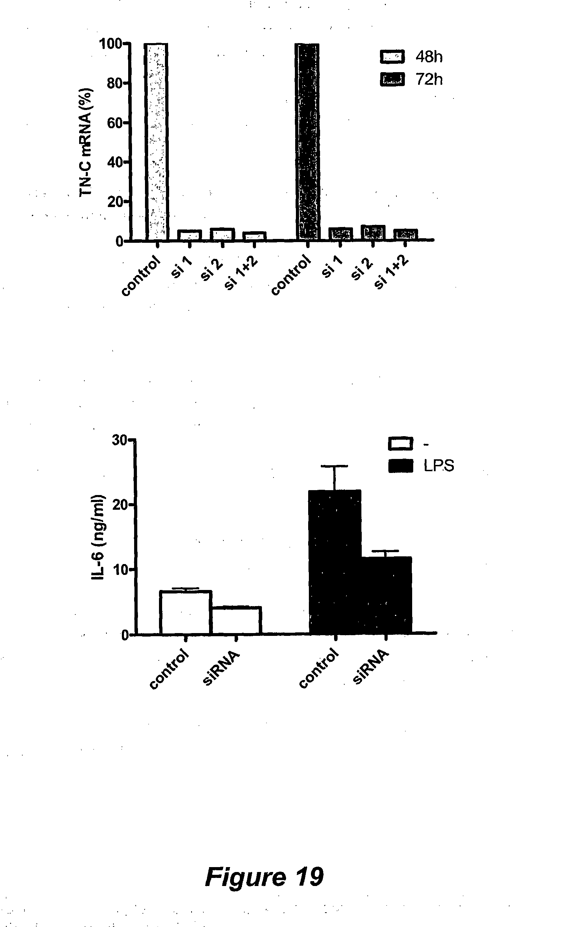

**Figure 19****. IL8 and TNF synthesis in response to tenascin-C targeted siRNAs.**
   Tenascin-C mRNA levels in RA fibroblasts transfected with luciferase specific siRNA (control), or with tenascin-C targeted siRNAs: oligo 1 (si 1), oligo 2 (si 2) or a combination of oligos 1+2 (si 1+2). IL6 synthesis in RA fibroblasts transfected with luciferase siRNA (control) or with a combination of tenascin-C targeted oligos 1+2 (siRNA) in the presence or absence of 10 ng/ml LPS for 24h.

### Example 1 -General methods

### Reagents

Zymosan, methylated BSA and Freund's complete adjuvant, anti-FLAG M2 antibody (mouse monoclonal antibody), blasticidin, and isotype control antibodies (Mouse IgG2a, IgG1) were from Sigma-Aldrich (Dorset, UK). Hypnorm was from VetaPharma Ltd. (Leeds, UK). The *Limulus amaebocyte* lysate assay was from Associates of Cape Cod (Liverpool, UK). Wild type human embryonic kidney (HEK293-EBNA) cells were from Invitrogen (Groningen, Netherlands). M-CSF and murine IL-1β were from PeproTech (Neuilly-Sur-Seine, France). DMEM, RPMI 1640, fetal bovine serum (FBS), penicillin/streptomycin, antibiotic-antimycotic solution PSA and β-Mercaptoethanol were from PAA Laboratories (Yeovil, UK). HEK293 cell lines stably expressing human TLR2 and TLR4/CD14/MD-2, polymyxin B, msbB LPS and the function blocking TLR2 (Clone: TL2.1 *Isotype:* Mouse IgG2a) and TLR4 antibodies (*Clone:* HTA125 *Isotype:* Mouse IgG2a) were from Invivogen (Calne, UK). Phenol-chloroform-purified *Escherichia coli* LPS (rough and smooth) and Pam3Cys-Ser-Lys4 (Pam3C) were from Alexis (Birmingham, UK). Murine TNF-α and IL-1 receptor antagonist (IL-1ra-IL-1F3) were from R&D Systems (Abingdon, UK). Function blocking anti-CD14 antibodies (*Isotype:* Mouse IgG1) were from Abcam (Cambridge, UK). Human and murine TNF-α, IL-6, and IL-8 ELISAs were from Pharmingen (Oxford, UK).

### Purification of full-length tenascin-C

To ensure that cytokine production was not attributed to bacterial contaminants such as LPS and LPS-associated molecules we purified recombinant full-length human tenascin-C from the conditioned medium of the mammalian cell line HEK293 transfected with his-tagged human tenascin-C in the pCEP-pu vector as described (Lange (2007)). tenascin-C was purified to homogeneity as described (Lange (2007) and determined to be free of LPS contamination using the *Limulus amaebocyte* lysate assay according to the manufacturer's instructions.

### Synthesis of recombinant proteins

Proteins corresponding to each domain of tenascin-C were synthesized (TA, EGF-L, various TNIII repeats and FBG) and purified. See Example 2.

### Measurement of LPS Contamination in Recombinant Proteins

To ascertain the levels of LPS in each recombinant protein the *Limulus amaebocyte* lysate assay was used according to the manufacturer's instructions (sensitivity ∼0.7 ± 0.5 pg LPS per mg protein). All recombinant proteins used in this study had levels of LPS that were less than 10pg/ml.

### Adenoviral Vectors and Their Propagation

Recombinant, replication-deficient adenoviral vectors encoding wild type MyD88 (AdMyD88wt), dominant-negative forms of MyD88 (AdMyD88dn) and the GFP control (AdGFP) were constructed in-house. A description of the synthesis of these viruses is in Andreakos (2004). All viruses used in this study are E1/E3 deleted, belong to the Ad5 serotype. Viruses were propagated in 293 human embryonic kidney cells, purified by ultracentrifugation through two cesium chloride gradients, and viral titers determined by plaque assay as previously described (Sacre (2007)).

### Animals

Homozygous tenascin-C deficient mice from the original stock described by Saga (1992) on a 129/sv an inbred strain of mice with a white bellied and agouti appearance background were provided by Prof. Charles French-Constant (University of Edinburgh, UK). Age matched congenic inbred wild type 129/sv mice were obtained from Charles River (Margate, UK). All tenascin-C deficient and wild type 129/sv mice were male and between 8-10 weeks of age at the time of experimentation.

Homozygous TLR2 and TLR4 deficient mice on a C57BL/6 background (an inbred strain of mice with a black coat) were obtained from B&K Universal (Hull, UK) Hoshino (1999) and Takeuchi (1999). Homozygous MyD88 deficient mice on a C57BL/6 background were provided by the Sanger Institute (Cambridge, UK). Age matched congenic inbred wild type C57B/L6 mice were obtained from Charles River (Margate, UK). For isolation of mouse embryo fibroblasts one female aged 8-10 weeks was mated with two males aged 8-10 weeks. For isolation of bone marrow derived macrophages mice were female and between 10-12 weeks of age at the time of experimentation.

All animals were fed standard rodent chow and water ad libitum, and were housed (<6 mice/cage) in sawdust-lined cages in an air-conditioned environment with 12-hour light/dark cycles. All animal procedures were approved by the institutional ethics committee.

### Statistical Methods

Mean, SD, SEM, and statistical tests were calculated using GraphPad version 3 (GraphPad Software Inc., San Diego, CA). Multiple group means were analyzed by one-way analysis of variance, followed by the Dunnett Multiple Comparisons test, where appropriate. Unpaired t-test was used for experiments involving only two groups.

### Example 2 - Synthesis of recombinant proteins

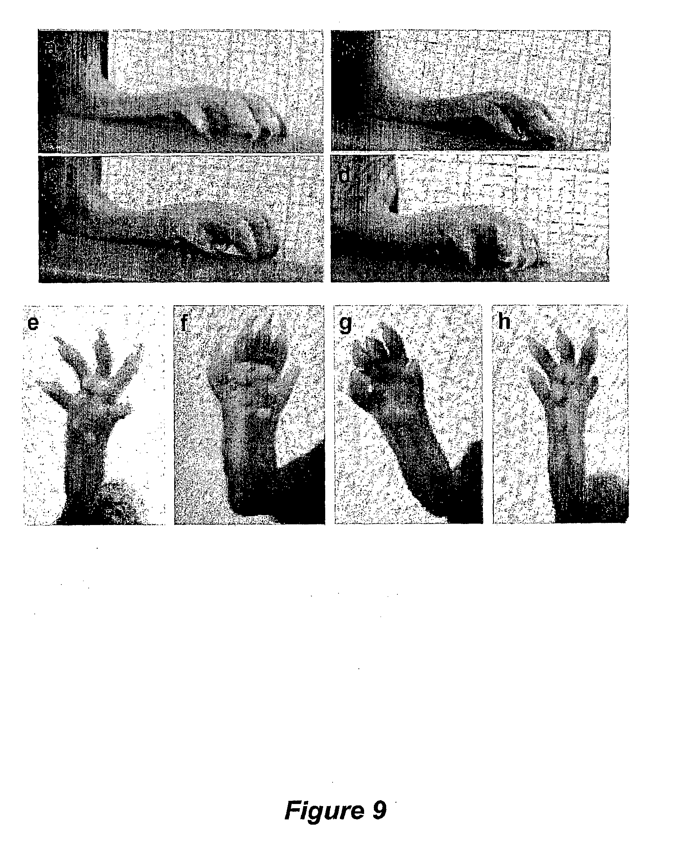

Proteins corresponding to each domain of tenascin-C were synthesized (TA, EGF-L, various TNIII repeats and FBG) and purified. The recombinant proteins synthesized are depicted in Figure 9.

### Reagents

Pfu Turbo polymerase was from Stratagene (Amsterdam, Netherlands). Easy mix 50 PCR tubes were from Molecular Bioproducts (Lutterworth, UK). RNeasy kits and Ni²⁺-NTA-agarose columns were from Qiagen (Crawley, UK). pCR Blunt vector, pCEP4 plasmid vector, human embryonic kidney (HEK293-EBNA) cells and 4-12% Bis-Tris gradient gels were from Invitrogen (Groningen, Netherlands). pET32b vector and BL21 (DE3) Rosetta cells were from Novagen (Kent, UK). HiTrap Q columns, HiTrap S columns, Sephacryl S500 HR column and heparin sepharose columns were from Amersham (Buckinghamshire, UK).

Restriction enzymes were obtained from New England BioLabs (Hitchin, UK). DMEM, fetal bovine serum (FBS) and penicillin/streptomycin were from PAA laboratories (Yeovil, UK). FuGENE6 transfection reagent was from Roche Applied Science (Basel, Switzerland).

Anti-FLAG M2 antibody (mouse monoclonal antibody), anti-FLAG M2-agarose, FLAG peptide were from Sigma-Aldrich (Dorset, UK). Anti-tetra-his antibody (mouse monoclonal antibody) was from Qiagen (Crawley, UK). Alkaline phosphatase-conjugated goat anti-(mouse IgG) IgG and Western Blue stabilized substrate for alkaline phosphatase were from Promega (Southampton, UK). Precision Protein Standards for SDS-PAGE were from BioRad (Hemel Hempstead, UK).

### Primer design

Domain boundaries were determined using alignments published in the human tenascin-C sequence (Siri (1991) accession number P24821 (Swiss-Prot)). To clone each domain we designed PCR primers where both the forward and reverse primers contained 18-21 bases corresponding to the 5' and 3' terminal sequences of the requisite coding sequence. The forward primer contained an Nde1 restriction site, followed by an N terminal his tag, immediately before the coding sequence. The final 3 bases of the Nde1 site form the ATC methionine initiation code. The reverse primer included a TTA stop codon immediately after the coding sequence, followed by a BamH1 or a Kpn1 site to allow unidirectional cloning into pET32b expression vectors.

**Table 1**

| Protein name | Forward primer |
|---|---|
| | Reverse primer |
| TA | FW: ATACATATGCATCATCATCATCATCATGGGGTCCTCAAG AAAGTCATCCGG |
| | RV: GCC***GGATCC***TTAGCCTGCTCCTGCAGTACATTG |
| EGF-L | PCR1 |
| | FW: ACAGTGGTACCACCATGGGGGCCATGGGGGCCATGACT CAGCTGTTG |
| | RV: CTTGTCATCGTCGTCCTTGTAGTCACCTTCGGTAGCGAG GGCAAG |
| | PCR2 |
| | FW: GACTAGAAGGACGACGATGACAAGTGCTGTCTCCAGCC TGCCAC |
| | RV: GACAGCGGATCCTTAATGATGATGATGATGATGTGAGCA GTCTTCTCCGCTGTAGC |
| TN1-5 | FW: ATACATATGCATCATCATCATCATCATGAGGTGTCTCCTCC CAAAGA |
| | RV: GCC***GGTACC***TTAAGTGGATGCCTTCACACGTGC |
| TN1-3 | FW: ATACATATGCATCATCATCATCATCATGAGGTGTCTCCTC CCAAAGA |
| | RV: GCC***GGTACC***TTATGTTGTGAAGGTCTCTTT GGC |
| TN3-5 | FW: ATACATATGCATCATCATCATCATCATCGCTTGGATGCC CCCAGCCAGAT |
| | RV: GCCGGTACCTTAAGTGGATGCCTTCACACGTGC |
| TN5-7 | FW: ATACATATGCATCATCATCATCATCATGAGTTGGACACG CCCAAGGAC |
| | RV: GCC***GGATCC***TTATGTTGTGAACTTGGCAGTGATGGTTG |
| TN6-8 | FW: ATACATATGCATCATCATCATCATCATGCCATGGGCTCCCC AAAGGAA |
| | RV: GCC***GGATCC***TTATGTGGTGAAGATGGTCTGGATCAT |
| FBG | FW: ATACATATGCATCATCATCATCATCATATTGGACTCCTGTAC CCCTTCC |
| | RV: GCC***GGATCC***TTATGCCCGTTTGCGCCTGCCT TCAA |

All primers above are written 5' to 3'. Flag sequences are in bold, His tags (CATCATCATCATCATCAT) are underlined, and restriction enzyme cleavage sites (CATATG = Nde1 site, GGATCC = BamH1, GGTACC = Kpn1 site) are in bold italics.

### PCR

PCR amplification was carried out using 10 pmol/µl of each primer, 1µg template, 5µl DMSO, and 1.25 units Pfu Turbo polymerase in a final volume of 25µl. This was added to buffer and dNTPs in Easy mix 50 tubes. The template used for all reactions was cDNA prepared from U87MG human glioma cells using RNA isolated with RNeasy kits. The reaction was cycled 40 times with denaturing, annealing and elongation temperatures of 95°C, 55-65°C (depending on melting temperature (Tm) of primers) and 72°C respectively.

### Cloning

PCR products were ligated into pCR Blunt vectors and sequenced to ensure no errors had been introduced by PCR. Clones were selected that had no errors or silent mutations. Inserts were then ligated into pET32b using Nde1 and BamH1 restriction sites engineered into primers (TN5-7 and TN6-8). Human tenascin-C has internal BamH1 sites within the TA domain (position 494) and TNIII2 (position 2509). TA and TN1-8 were therefore cloned using the Nde1 site in the FW primer and the Kpn1 site in the cloning site of pCRBlunt. Human tenascin-C contains no internal Kpn1 sites. TN1-5, TN1-3 and TN3-5 were cloned using Nde1 and Kpn1 sites in the primers. FBG contains an internal Nde1 site (position 6439) and was therefore cloned using a two step ligation of Nde1 and BamH1 digestion, followed by Nde1 digestion. (Positions refer to sites within the full length nucleotide sequence of tenascin-C, given in figure 14)

### Bacterial growth, induction and lysis

The plasmids were transformed into BL21 (DE3) Rosetta cells, cultured in 3L of Luria-Bertani medium containing 50 µg/ml carbenicillin and induced with 1 mM isopropyl-β-D-thiogalactopyranoside. After 3 hours, the cells were harvested by centrifugation at 4,000 rpm for 20 min, washed twice with ice-cold wash buffer (50 mM Tris-HCl, pH 8.0, 100 mM NaCl, and 1 mM EDTA), and lysed with a French press. Inclusion bodies were collected by centrifugation at 12,000 rpm for 20 min at 4 °C. With the exception of TA and FBG the proteins were located entirely in the supernatant. Recombinant TA and FBG proteins were extracted from inclusion bodies with 6 M guanidine hydrochloride, 50 mM Tris-HCl, pH 8.0, and 10 mM β-mercaptoethanol at room temperature with constant stirring for 2 hours.

### Purification of bacterial proteins

The solution containing recombinant protein was applied to a Ni²⁺-NTA-agarose column and washed with 50 mM Tris-HCl, pH 8.0 containing 20 mM imidazole. The column was subsequently washed with 50 mM Tris-HCl, pH 8.0 and the protein was eluted with 50 mM Tris-HCl, pH 8.0 containing 60 mM imidazole. For TA and FBG each washing and elution buffer contained 6 M guanidine hydrochloride. Following Ni chromatography TA and FBG required no subsequent purification. TN1-3 and TN6-8 were further purified by anion exchange chromatography using a HiTrap Q column, TN1-5, TN3-5 and TN5-7 by cation exchange chromatography using a HiTrap S column, and TN1-8 using a HiTrap S column followed by gel filtration using a Sephacryl S500 HR column.

### Refolding of insoluble proteins

TA and FBG were refolded by diluting to 20 µg/ml with 50 mM Tris-HCl, pH 8.0 containing 6 M guanidine hydrochloride and then treating with 20 mM cystamine with stirring for 16 hours at 4°C. The solution was then dialyzed twice against 15 volumes of 50 mM Tris-HCl, pH 8.0 containing 150 mM NaCl, 10 mM CaCl₂, 5mM β-mercaptoethanol, and 1 mM 2-hydroxyethyl disulfide for 24 hours at 4 °C, twice against 20 mM Tris-HCl, pH 8.0 for 8 hours at 4 °C and then centrifuged at 12,000 rpm for 30 min at 4 °C. Refolding was assessed by size shifts using SDS PAGE under reducing and non reducing conditions. Protein activity was confirmed by TA domain polymerization and FBG binding to heparin sepharose columns.

### Synthesis of EGF-L domain using mammalian cells

Initial attempts to express and purify the EGF-L repeats region using an *E.coli* expression system were unsuccessful. This is most likely to be attributable to difficulty in achieving protein folding due to a total of 91 cysteines in this region. Therefore, the EGF-like domains of TN-C were expressed using HEK293 cells.

Two PCR reactions were carried out. The first PCR product consisted of a restriction enzyme *Kpn*I site, a Kozak sequence followed by the TN-C signal sequence. The second PCR product consisted of a FLAG peptide, the EGF-like domain sequence, followed by a histidine tag and a BamH1 restriction enzyme sequence.

The two PCR products were ligated together as described by Ho (1989). PCR reactions were carried out as described above. The entire construct was cloned into the PCR blunt vector and sequenced. It was then subcloned into the pCEP4 vector. The DNA was transfected into HEK293 cells using Fugene and cells were selected for hygromycin resistance (200 µg/ml) in Dulbecco's modified Eagle's medium (DMEM) containing 10% (v/v) fetal calf serum, penicillin (100 units/ml) and streptomycin (100 units/ml). 2 litres conditioned medium (collected after cells have been cultured in medium) from stably transfected cells was collected and pooled. The pooled conditioned medium (2 litres) was centrifuged at 3000 rpm to separate cell debris from the medium.

The medium was then applied to an anti-FLAG column. Material was collected in 50 ml fractions for the flow-through. The column was washed with 10 column volumes of 1 M NaCl, 50 mM Tris-HCl, pH 7.5 and then washed with 10 column volumes of 60% isopropanol to ensure removal of LPS. The column was then washed with 50 mM Tris-HCl buffer, pH 7.5 and finally the protein was eluted using 200 µg/ml FLAG peptide in 50 mM Tris-HCl buffer, pH 7.5.

### Analysis of protein purity

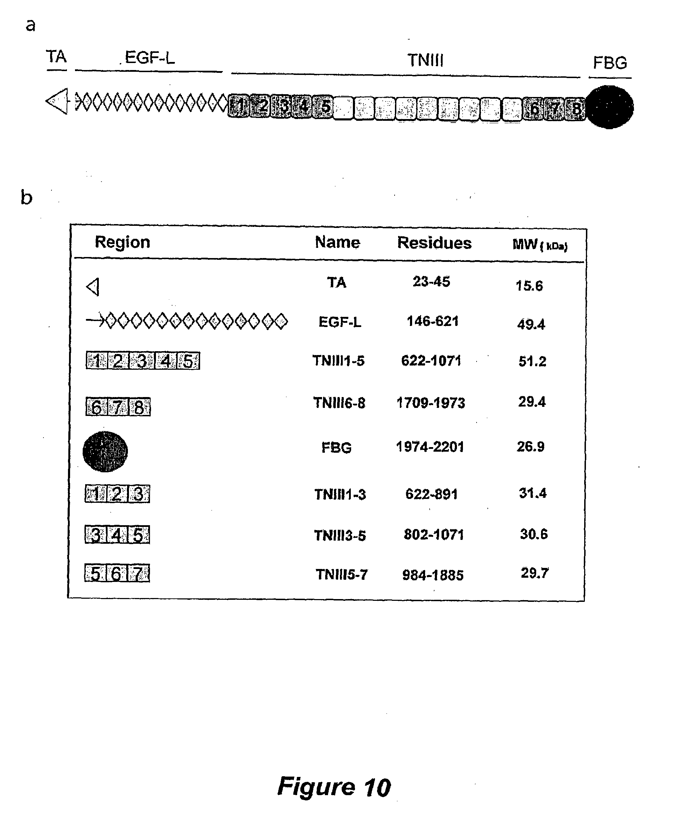

Each protein was dialysed against 1000 volumes of 150 mM NaCl and 50mM Tris pH 7.5. Protein purity was analyzed by SDSPAGE under reducing conditions. To do this 1 µg of each purified recombinant protein was run on a 4-12% Bis-Tris gradient gel and the gel was subsequently silver stained to demonstrate a single band (figure 10). Western blotting analyses were also carried out. Proteins separated by SDS-PAGE were electrotransferred to polyvinylidene difluoride membranes. The membranes were blocked with 5% BSA in Tris-buffered saline and then incubated with primary antibodies recognizing FLAG M2 (1:2000 dilution)(EGF-L) or tetra-his antibodies (1:2000)(all other proteins). The blot was then incubated with secondary antibody conjugated to alkaline phosphatase and the protein bands visualized using Western Blue stabilized substrate whereby the gels show a single specific band recognised by each antibody at the expected Mw (not shown)

### Example 3 - Animal models

### Zymosan-induced arthritis

Zymosan-induced arthritis (ZIA) was induced in tenascin-C deficient and wild type mice by injection of zymosan (*Saccharomyces cerevisiae*), as described in Keystone (1977). Zymosan was prepared by dissolving 15 mg of zymosan in 1ml of sterile PBS. The solution was boiled twice and sonicated. Mice were anesthetized by intraperitoneal injection of 150 µl of Hypnorm diluted 1:10 in sterile water, then injected with zymosan (10 µl) into the right footpad (d=0).

Control mice received an injection of 10 µl PBS alone or were not injected. For macroscopic assessment of arthritis, the thickness of each hind paw was measured daily with microcalipers (Kroeplin, Schluchlem, Germany) and the diameter expressed as an average for each inflamed hind paw per mouse.

Following completion of the experiment (day=4), mice were euthanized and hind paws fixed in 10% (v/v) buffered formalin, decalcified with 10% EDTA and processed to paraffin.

### Antigen-induced arthritis

Antigen-induced arthritis (AIA) was induced in tenascin-C-deficient and wild-type mice as described previously by Brackertz (1977). Briefly, at day 0 mice were anesthetized by intraperitoneal injection of 150 µl of Hypnorm diluted 1:10 in sterile water, then immunized with 200 µg of methylated BSA. mBSA was emulsified in 0.2 ml of Freund's complete adjuvant and injected intra-dermally at the base of the tail.

At day 7, arthritis was induced by intra-articular injection of mBSA (100 µg in 10 µl of sterile PBS) into the right knee joint using a sterile 33-gauge microcannula. Control mice received an injection of 10 l PBS alone or were not injected.

On day 14, mice were euthanized, the knee joints were excised and fixed in 10% (volume/volume) buffered formalin, decalcified, with 10% EDTA and processed to paraffin.

### Injection of FBG

Wild type mice were anesthetized by intraperitoneal injection of 150 µl of Hypnorm diluted 1:10 in sterile water and then injected with 100 ng, 1 or 3 µg FBG in 10 µl of sterile PBS into the right knee joint using a sterile 33-gauge microcannula. Control mice received an injection of 10 µl PBS alone or were not injected.

On days 3 and 7, mice were euthanized, the knee joints were excised and fixed in 10% (volume/volume) buffered formalin, decalcified, with 10% EDTA and processed to paraffin.

### Histology of knee joints

Coronal tissue sections (4 µm) were cut at 7 depths throughout the joint; 80 µm apart and stained with hematoxylin and eosin or Safranin-O to assess joint pathology. Histopathologic changes were scored using the following parameters as described in Van Lent (2006).

Inflammation (the influx of inflammatory cells into synovium (infiltrate) and the joint cavity (exudates), was graded using an arbitrary scale from 0 (no inflammation) to 3 (severe inflammation). Chondrocyte death was determined as the percentage of cartilage area containing empty lacunae in relation to the total area. Cartilage surface erosion was determined as the amount of cartilage lost in relation to the total cartilage area. Bone destruction was determined in 10 different areas of the total knee joint section. Destruction was graded on a scale of 0 (no damage) to 3 (complete loss of bone structure). Histological analysis was performed by an investigator who was blinded to the experimental groups. The mean score for each animal in an experimental group was calculated by averaging the histopathologic scores in at least 5 section depths per joint.

### Results

### Zymosan induced joint inflammation is not sustained in tenascin-C deficient mice

Zymosan injection into the footpad was used to induce acute synovitis in mice. Wild type mice exhibited rapid paw swelling reaching maximal paw diameter by 24 hours (2.56mm, an increase of 62% of the starting paw diameter). This was maintained for a further 24 hours. After 2 days paw diameter decreased but paws remained swollen by 4 days (2.08mm, an increase of 32%) (figure 1a). tenascin-C deficient mice exhibited a similar degree of paw swelling to wild type mice 24 hours post injection (2.41 mm, an increase of 57% of starting paw diameter). However, swelling in the tenascin-C null mice subsided faster than in the wild type mice; paw diameter was significantly reduced at 2 days and had declined to 1.7mm (an increase of only 11%) by 4 days (figure 1a). By day 4 post injection the paws of wild type mice were still visibly swollen and red, whereas the paws of tenascin-C null mice were not visibly swollen or red and resembled non-injected paws (Figure 9).

This difference was reflected histologically at 4 days. The synovia of wild type mice were significantly inflamed and exhibited cellular infiltration and cartilage proteoglycan loss was observed (Figure 1b, c). In contrast, the synovium of tenascin-C deficient mice exhibited no synovitis, cellular infiltrate or cartilage proteoglycan loss (figure 1d, e) and resembled the joints of sham injected and non injected mice (not shown). Quantification of joint inflammation revealed whilst there was little exudate (cellular mass in the joint cavity) in either wild type or tenascin-C null mice, levels of infiltrate (cellular mass in the synovial layer) were significantly reduced in tenascin-C null mice (figure 1f). No erosion of cartilage or bone occurred in mice of either genotype (not shown), however a low level of chondrocyte death occurred in wild type mice, that was not observed in tenascin-C null mice (figure 1g). Thus tenascin-C expression appears to promote the maintenance of acute inflammation.

### Tenascin-C null mice are protected from persistent inflammation and structural damage during antigen induced arthritis

To determine whether tenascin-C also contributes to more destructive inflammatory joint disease, erosive arthritis was induced by intra-articular injection of mBSA into the knee joint following immunization with mBSA. This model involves both cellular and humoral immune responses and induces pathological changes similar to human RA (Brackertz (1977)). Injection of mBSA induced a similar inflammatory response in both tenascin-C null and wild type mice. Cell infiltration and synovial thickening is apparent by 24 hours in mice of both genotypes (figure 2c-f, h,i) compared to sham injected (figure 2a, b, g) or non injected (not shown) mice.

However, this does not persist in tenascin-C null mice as it does the wild type mice. By 3 days post injection wild type mice exhibit increased inflammation of the meniscus and capsule, synovial hyperplasia, cells and fibrin deposits in the joint space, pannus formation and localized cartilage proteoglycan loss (figure 3a, b, f). In contrast, by 3 days in tenascin-C null mice inflammation is limited to the capsule, synovial inflammation has subsided and there are no fibrin/cell aggregates present in the joint space, no pannus formation and no cartilage proteoglycan loss (figure 3c, d, e).

By 7 days wild type mice exhibited persistent inflammatory cell infiltration and joint space exudate, extensive synovitis and pannus formation and destruction of articular cartilage and bone erosion (figure 4a, b). Sham injected knees and knees from mice that had undergone no injection were healthy and exhibited no inflammation or joint destruction (not shown). tenascin-C deficient mice also had healthy joints that exhibited only mild inflammatory cell infiltration, with no joint space exudate, synovitis, pannus formation, destruction of articular cartilage or bone erosion (figure 4c, d). Joints from tenascin-C deficient mice that had been sham injected and or that had undergone no injection were also healthy (not shown).

These histological data are reflected upon scoring of joint disease as described in materials and methods. Levels of cellular infiltrate and exudate observed in both wild type mice and tenascin-C null mice 24 hours post injection were not significantly different. However, whilst cellular mass continued to increase in wild type mice over time, this response was attenuated in tenascin-C null mice and cell numbers in the joint decreased over time (figure 4e). Increasingly high levels of chondrocyte death occurred in the cartilage of wild type mice over time, but no significant death was observed in tenascin-C null mice (figure 4f). No cartilage surface erosion and bone erosion was evident in wild type mice at 24 hours or 3 days (not shown) but significant tissue destruction had occurred by 7 days. In contrast tenascin-C null mice exhibited no tissue destruction at 24 hours, 3 days (not shown) or 7 days (figure 4f). These data indicate that whilst the initiation of joint inflammation (cell influx into the synovium and joint space) is unaffected in tenascin-C null mice, unlike in wild type mice disease does not progress to tissue destruction and cell death. These results demonstrate that expression of tenascin-C is required for persistent synovial inflammation and joint destruction in this model.

### Example 4 - Cell culture

### Patient Specimens -

Human monocytes were isolated from peripheral blood (London Blood Bank) and macrophages were derived from monocytes after differentiation for 4 days with 100 ng/ml of M-CSF as previously described (Foxwell (1998)).

RA membrane cells (representing a mixed population of all synovial cell types) were isolated from synovial membranes obtained from patients undergoing joint replacement surgery as previously described (Brennan(1989)). RA synovial fibroblasts were isolated from the mixed population of RA membrane cells as previously described (Brennan(1989)). The study was approved by the local Trust ethics committee (Riverside NHS Research Committee), and waste tissue (synovium after joint replacement surgery) was obtained only after receiving signed informed consent from the patient and anonymyzing the tissue to protect patient identity.

Immediately after isolation, RA membrane cells and macrophages were cultured at 1x10⁵ cells/well in RPMI 1640 containing 10% (v/v) FBS and 100 U/ml (Units/ml) penicillin/streptomycin in 96-well tissue culture plates for 24 hours before stimulation. Synovial fibroblasts (used only at either passage number 2 or 3) were cultured at 1x10⁴ cells/well in DMEM containing 10% (v/v) FBS and 100 U/ml penicillin/streptomycin in 96-well tissue culture plates for 24 hours before stimulation.

### Mouse embryonic fibroblasts (MEFs) and bone marrow derived macrophages (BMDMs)

MEFs express high levels of mRNA of all 9 murine TLRs and are specifically and highly responsive to TLR ligand activation. MEFs from mice with targeted deletions of TLR2, TLR4 and MyD88 demonstrate profound defects in their IL-6 response to specific ligands (Kurt-Jones (2004)). MEFs were isolated from d13 embryos harvested from age-matched, pregnant female wild type, TLR2, TLR4 and null mice (as described in Todaro (1963)). Fibroblasts were cultured at 2x10⁴ cells/well in DMEM containing 10% (v/v) FBS and 100 U/ml penicillin/streptomycin in 96-well tissue culture plates for 24 hours before stimulation.

BMDMs were derived by aspirating the femurs of age matched female wild type, TLR2 and TLR4 null mice as described in Butler (1999)) and culturing the cells for 7 days in DMEM, 20% (v/v) FBS, 10ml/L (v/v) antibiotic-antimycotic solution PSA, 50µM β-Mercaptoethanol and 10ng/ml M-CSF. Macrophages were then cultured at 1x10⁵ cells/well in DMEM, 20% (v/v) FBS, 10ml/L (v/v) antibiotic-antimycotic solution PSA, 50µM β-Mercaptoethanol in 96-well tissue culture plates for 24 hours before stimulation.

### HEK293 cell lines

HEK293 cell lines expressing TLR2 and TLR4/CD14/MD-2 were cultured at 1x10⁴ cells/well in DMEM containing 10% (v/v) FBS and 10 µg/ml blasticidin in 96-well tissue culture plates for 24 hours before stimulation.

### Cell stimulation and assessment of cytokine synthesis

Cells were incubated for 24 hours at 37°C with the indicated doses of tenascin-C and recombinant tenascin-C fragments (1.0 µM - 1.0 nM). Cells were also stimulated where indicated with LPS (1 ng/ml for human macrophages, 10ng/ml for human fibroblasts, RA membrane cells and HEKs, 100ng/ml for MEFS and BMDMs and 10ng/ml for HEKS), PAM3 (10 ng/ml for human macrophages, human fibroblasts, and HEKs, 100ng/ml for MEFs and BMDMs), murine IL-1 (5ng/ml for MEFS) and murine TNF-α (100ng/ml for MEFS). Unless specifically stated otherwise rough LPS was used for *in vitro* studies.

For adenoviral gene transfer experiments, human RA synovial fibroblasts were incubated with adenoviral vectors at a multiplicity of infection of 100, washed after 2 hours, cultured in complete medium for 24 hours, then stimulated for 24 hours, after which time supernatants were collected.

Where stated, cells were pre-incubated with 10µg/ml anti-CD14 antibody, 10µg/ml IL1 receptor antagonist, 10µg/ml anti-TLR2 antibody, 25µg/ml anti-TLR4 antibody, 10 or 25µg/ml isotype control antibody, 25µg/ml polymyxin B, or 1µg/ml msbB LPS, for 30 minutes at 37°C before stimulation. Where stated, recombinant tenascin-C and FBG, and LPS were boiled for 15 minutes before addition to cells

In all cases, viability of the cells was not significantly affected throughout the experimental time period when examined by the MTT cell viability assay (Sigma, Poole, UK).

Supernatants were subsequently examined for the presence of the cytokines TNF-α, IL-6, and IL-8 by enzyme-linked immunosorbent assay (ELISA) according to the manufacturer's instructions. Absorbance was read on a spectrophotometric ELISA plate reader (Labsystems Multiscan Biochromic, Vantaa, Finland) and analyzed using the Ascent software program (Thermo Labsystems, Altrincham, UK).

### Results

### Tenascin-C induces TNF-α, IL-6 and IL-8 synthesis in primary human RA synovial fibroblasts and macrophages

We next investigated whether tenascin-C might activate the innate immune response. tenascin-C was used to stimulate primary human macrophages and RA synovial fibroblasts and the production of the pro-inflammatory cytokines TNF-α, IL-6 and IL-8 examined. The bacterial cell wall component LPS was used as a positive control. tenascin-C induced a cell type specific cytokine profile which was significantly different from LPS. It dose dependently stimulated the production of TNF-α, IL-6 and IL-8 in human macrophages (figure 5a). However, tenascin-C only induced IL-6 synthesis in synovial fibroblasts, whereas LPS induced both IL-6 and IL-8 (figure 5b). Neither LPS nor tenascin-C induced TNF-α synthesis in fibroblasts (data not shown). tenascin-C stimulation of IL-6 (Figure 5c), IL-8 and TNF-α by human macrophages and IL-6 by synovial fibroblasts (not shown) was heat sensitive and unaffected by the LPS inhibitor, polymyxin B. Together these results provide strong evidence that cytokine induction by tenascin-C is not due to LPS contamination.

### The fibrinogen-like globe (FBG) mediates tenascin-C activation of cells.

Tenascin-C is a large hexameric molecule, each domain of which binds to different cell surface receptors (reviewed in Orend (2005)). Understanding the mechanism of action of tenascin-C will require identification of which domain(s) are critical for promoting cytokine production. We synthesized recombinant proteins comprising different domains of the molecule (figure 10). Each domain was made in *E.coli*, purified (figure 11), and found to contain <10 pg/ml LPS by subjecting neat protein to the *Limulus amaebocyte* lysate assay. Only one domain of tenascin-C was active. The fibrinogen-like globe (FBG) stimulated TNF-α synthesis in human macrophages (Figure 6a), IL-6 and IL-8 synthesis in human macrophages (not shown) and IL-6 in RA synovial fibroblasts (not shown) to an equal extent to full-length tenascin-C. Like full-length tenascin-C, FBG did not induce IL-8 synthesis in RA synovial fibroblasts where LPS did (data not shown). FBG induced cytokine synthesis was also heat sensitive and unaffected by polymyxin B (data not shown).

### The FBG domain of tenascin-C induces cytokine production in human RA synovium and joint inflammation in mice.

We investigated whether FBG could promote expression of inflammatory cytokines in synovial membranes from RA patients. This tissue model of RA (comprising a mixed population of all synovial cell types) spontaneously produces high levels of IL-6, IL-8 and TNF-α (Brennan (1989)) (figure 6b). FBG further enhanced synthesis of all these cytokines (figure 6b). To determine whether FBG could induce inflammation *in vivo*, wild type mice were injected intra-articularly with FBG. We observed a transient and dose dependent stimulation of joint inflammation. No inflammation or proteoglycan loss occurred in non-injected mice or in mice injected with PBS (figure 6c-e) or 100ng FBG (data not shown). In mice injected with 1 µg FBG inflammatory cell infiltration (figure 6f), mild synovitis, pannus formation (figure 6g) and proteoglycan loss (figure 6h) was observed. A similar response was seen in mice injected with 3 µg FBG (data not shown). Upon histological quantification, high levels of cellular infiltrate and exudate and chondrocyte death were observed in mice injected with FBG, together with a modest amount of cartilage surface erosion and bone damage (figure 6i).

### FBG mediated cytokine synthesis is dependent on Myd88

Many DAMPs, including fibrinogen (Smiley (2001)), have been shown to stimulate the innate immune response by activation of TLRs. Therefore, we investigated whether TLRs might also mediate tenascin-C induced cytokine production. Myeloid differentiation factor 88 (MyD88) is required for signalling by all TLRs, except TLR3 (O'Neill (2008)). Infection of synovial fibroblasts with adenovirus expressing dominant negative MyD88, but not GFP control virus, abolished FBG induction of IL-6 (figure 7a). These data suggest that FBG induced inflammation is dependent on functional MyD88. This effect of FBG did not appear to be mediated by IL-1 as addition of IL-1 receptor antagonist did not inhibit induction of cytokines (data not shown). To confirm that FBG action is MyD88 dependent we demonstrated that FBG does not stimulate cytokine synthesis in embryonic fibroblasts isolated from mice with targeted deletions in the MyD88 gene. The TLR2 ligand PAM3, TLR4 ligand LPS and IL-1 all signal via MyD88. Stimulation with these was also abolished in MEFs from deficient mice. However, TNF-α, which does not signal via MyD88, was unaffected (figure 7b). Re-transfection of wild type MyD88 restored the responsiveness of these cells to FBG, PAM3, LPS and IL-1 (data not shown).

### FBG signals via TLR4

TLRs exhibit specificity for endogenous ligands; proteins are recognised by one or both of TLR2 and 4 (reviewed in O'Neill (2008)). Neutralising antibodies to TLR4 inhibited both FBG and LPS induced IL-6, IL-8 and TNF-α synthesis in human macrophages and IL-6 synthesis in RA synovial fibroblasts but had no effect on the function of the TLR2 ligand, PAM3. Antibodies to TLR2 inhibited PAM3 mediated cytokine synthesis but had no effect on LPS or FBG induced cytokine synthesis. Isotype matched controls had no effect on cytokine synthesis induced by any ligand (TNF-α synthesis by human macrophages is shown in figure 8a). To confirm that FBG action is TLR4 dependent we demonstrated that FBG does not stimulate cytokine synthesis in embryonic fibroblasts or macrophages isolated from mice with targeted deletions in the TLR4 gene. FBG mediated cytokine synthesis was unaffected in embryonic fibroblasts or macrophages isolated from mice with targeted deletions in the TLR2 gene. Cells isolated from TLR2 deficient mice were unresponsive to PAM3 but responsive to LPS and IL-1. Cells isolated from TLR4 deficient mice were unresponsive to LPS but did respond to PAM3 and IL-1 (figure 8b, c). In addition, expression of TLR4 was required for the arthritogenic action of FBG in vivo; FBG was able to induce joint inflammation in TLR2 null mice but not in TLR4 null mice (figure 12).

### Different co-receptor requirements for FBG and LPS

LPS signalling via TLR4 is mediated by a receptor complex including the soluble protein MD-2 and GPI-linked cell surface or soluble CD14 (reviewed in Fitzgerald (2004)). We next examined whether CD14 and MD-2 are required for FBG activation of TLR4. As a positive control here we examined the activity of smooth glycosylated LPS which requires both MD-2 and CD14 (Jiang (2005)). LPS mediated IL-6, IL-8 and TNF-α synthesis by human macrophages and IL-6 synthesis by RA synovial fibroblasts was inhibited by anti-CD14 antibodies and an antagonistic LPS derived from the msbB mutant E.coli which competes for LPS binding to MD-2 (Coats(2007)). Conversely, both PAM3, which does not require these co-receptors for activation of TLR2, and FBG-mediated cytokine synthesis was unaffected by anti CD14 antibodies or msbB mutant LPS (figure 8d shows TNF-α synthesis by human macrophages). These data suggest that neither CD14 nor MD-2 is required for FBG mediated cytokine synthesis. Therefore, whilst LPS and FBG both signal via activation of TLR4, they may have different co-receptor requirements.

### Example 5 - Inhibition of Tenascin-C action and synthesis in human tissue

This example studies the effect of (1) prevention ofn the pro-inflammatory action of tenascin-C and (2) inhibition of tenascin-C expression in the human RA synovium.

### Methods

### Peptide synthesis

Nine overlapping peptides comprising the entire FBG domain (table 2) were synthesized by Biogenes, Germany. Peptides were cleaved at room temperature (cleavage mixture: 90% trifluoroacetate, 5% thioanisol, 3% ethanedithiol, 2% anisole), purified by reverse phase high performance liquid chromatography, and characterized by MALDI TOF mass spectral analysis. The purity of the peptides was >85% as determined high performance liquid chromatography.

The facility was unable to synthesize peptide 7, presumably due to the formation of secondary structure that prevented elongation of the peptide chain (as previously reported (LaFleur (1997)).

**Table 2. Overlapping peptides that span the entire FBG domain of human tenascin-C**

| **Peptide #** | **Amino acid sequence** |
|---|---|
| 1 | TIGLLYPFPKDCSQAMLNGDTTSGLYTIYL |
| 2 | YTIYLNGDKAEALEVFCDMTSDGGGWIVFL |
| 3 | WIVFLRRKNGRENFYQNWKAYAAGFGDRRE |
| 4 | GDRREEFWLGLDNLNKITAQGQYELRVD |
| 5 | ELRVDLRDHGETAFAVYDKFSVGDAKTRYK |
| 6 | KTRYKLKVEGYSGTAGDSMAYHNGRSFST |
| 7 | RSFSTFDKDTDSAITNCALSYKGAFWYRN |
| 8 | WYRNCHRVNLMGRYGDNNHSQGVNWFHWKG |
| 9 | FHWKGHEHSIQFAEMKLRPSNFRNLEGRRKRA |

### Patient specimens and cell culture

RA membrane cells (representing a mixed population of all synovial cell types) were isolated from synovial membranes obtained from patients undergoing joint replacement surgery (Brennan (1989)). Synovial membrane tissue was digested in RPMI 1640 (GIBCO) containing 5% fetal calf serum (FCS) (GIBCO), 5 mg/ml collagenase type IV (Sigma) and 0 15 mg/ml DNAse type I (Sigma) and incubated at 37°C for 2 h.

After incubation the tissue was pipetted through a nylon mesh into a sterile beaker. The cells were then washed three times in complete medium (RPMI 1640 supplemented with 10% FCS). RA synovial fibroblasts were isolated from the mixed population of RA membrane cells by selection in DMEM (Bio-Whittaker) supplemented with 10% FBS, 1 µM glutamine, 100 U/ml penicillin, and streptomycin. Human monocytes were isolated from peripheral blood (London Blood Bank) and macrophages were derived from monocytes after differentiation for 4 days with 100 ng/ml of M-CSF.

The study was approved by the local Trust ethics committee, and waste tissue (synovium after joint replacement surgery) was obtained only after receiving signed informed consent from the patient and anonymyzing the tissue to protect patient identity.

### Cell stimulation and assessment of cytokine synthesis

Immediately after isolation, RA membrane cells were cultured at 1x10⁵ cells/well in RPMI 1640 containing 10% (v/v) FBS and 100 U/ml penicillin/streptomycin in 96-well tissue culture plates. Cells were incubated for 24h at 37°C with no addition, buffer control (PBS, 1% BSA, 0.01% NaN₃), or with 25 µm, 100 µM or 250 µM of each FBG spanning peptide.

Synovial fibroblasts (used only at either passage number 2 or 3) were seeded at a concentration of 5 × 10⁴ cells in a 3.5-cm dish. siRNA was transfected at a final concentration of 10 nM using Lipofectamine 2000 (Invitrogen) for 4 h in serum-free OptiMEM I. Two different siRNAs against human tenascin-C were used (s7069 and s229491) (Applied Biosystems).

siRNA sequences of s7069 are: (sense 5' CGCGAGAACUUCUACCAAAtt 3', antisense 5' UUUGGUAGAAGUUCUCGCGtc 3') and of s229491 are (5' GGAAUAUGAAUAAAGAAGAtt 3', antisense 5' UCUUCUUUAUUCAUAUUCCgg 3'). siRNA against luciferase (Dharmacon) was transfected as a non-targeting control.

Four hours after transfection, medium was changed with pre-equilibrated Dulbecco's modified Eagle's medium containing 10% FBS (v/v) and cells were incubated for a further 48h and 72h. Cells were then stimulated with 10 ng/ml LPS for 24h at 37°C. Tenascin-C mRNA and protein levels were quantitated by PCR and western blotting respectively. Total RNA was extracted from cells using a QiaAmp RNA Blood mini kit (Qiagen, Germany). cDNA was synthesised from equivalent amounts of total RNA using SuperScript® III Reverse Transcriptase (Invitrogen) and 18-mer oligo dTs (Eurofins MWG Operon).

Gene expression was analysed by delta-delta ct methods based on quantitative real-time PCR with TaqMan primer set human tenascin-C (Hs01115663-m1) and human ribosomal protein endogenous control (RPLPO) (4310879E) (Applied Biosystems) in a Corbett Rotor-gene 6000 machine (Corbett Research Ltd). Tenascin-C protein was detected in cell supernatants and cell lysates by by SDS PAGE and western blotting using antibody MAB1908 (Millipore).

Macrophages were cultured at 1x10⁵ cells/well in RPMI 1640 containing 5% (v/v) FBS and 100 U/ml penicillin/streptomycin in 96-well tissue culture plates for 24h before stimulation. Cells were incubated for 24h at 37°C with no addition, 1.0 µM FBG, 1 ng/ml LPS or 1 or 20 µM FBG peptide. Where stated, cells were pre-incubated with 20 µM FBG peptides for 15 min.

The viability of the cells was not significantly affected throughout the experimental time period when examined by the MTT cell viability assay (Sigma, Poole, UK). Supernatants were examined for the presence of the cytokines TNF-α, IL-6, and IL-8 by enzyme-linked immunosorbent assay (ELISA) according to the manufacturer's instructions (R&D systems). Absorbance was read on a spectrophotometric ELISA plate reader (Labsystems Multiscan Biochromic, Vantaa, Finland) and analyzed using the Ascent software program (Thermo Labsystems, Altrincham, UK).

### Statistical Methods

Mean, SD and SEM were calculated using GraphPad (GraphPad Software Inc., San Diego, CA).

### Results

### Blockade of cytokine synthesis in RA membrane cultures by specific FBG peptides

The approach of peptide inhibition has been used successfully to pinpoint the αvβ3 integrin binding site in the FBG domain of tenascin-C and to prevent cell adhesion in response to this domain of tenascin-C (Lafleur (1997) and Yokoyama (2000)).

We synthesized a series of 8 overlapping peptides of -30 amino acids that span the entire sequence of FBG (Table 2). Peptides were tested for the ability to block spontaneous cytokine synthesis in RA synovial membrane cultures. TNF and IL8 synthesis was inhibited by peptides 3 and 8, but not by any other peptide (TNF shown in figure 15). Peptides 3 and 8 dose dependently inhibited cytokine synthesis with the highest concentrations achieving 95% and 56% inhibition respectively (figure 16). Whilst peptide 5 had no effect on TNF synthesis, it dose dependently blocked IL8 synthesis in RA membrane cells with a maximal inhibition of 81 % (figure 16).

To map the active domain within FBG responsible for inducing cytokine production we stimulated primary human macrophages with each FBG peptide. Peptides 1, 5 and 6 all induced cytokine synthesis in a dose dependent manner. (figure 17).

To determine if any peptide could block FBG induced cytokine synthesis in human macrophages, cells were pre-incubated with each FBG peptide before stimulation with either whole FBG or LPS. Peptide 5 specifically blocked FBG mediated cytokine synthesis, whilst peptide 8 blocked cytokine synthesis in response to both LPS and FBG (figure 18).

Peptide 8 therefore non-specifically blocks cytokine production induced by any stimuli. This domain is the integrin binding domain of FBG that mediates cell adhesion and thus may be acting to prevent cell attachment to tissue culture plates. Peptide 5 specifically blocks FBG-induced cytokine synthesis suggesting that targeting this domain may be useful in preventing tenascin-C induced inflammation.

### Silencing tenascin-C gene expression inhibits cytokine synthesis in RA synovial fibroblasts

Examination of the effect of inhibiting tenascin-C expression in the human RA synovium has identified synovial fibroblasts as the major source of tenascin-C in RA (figure 1 C) (in Goh 2010).

siRNA mediated knockdown of tenascin-C expression in these cells has been shown with a maximal efficiency between 94-96% (figure 19). In cells transfected with tenascin-C siRNA, both the basal level of cytokine synthesis and LPS induced cytokine production was inhibited by 38% and 44% respectively compared to control cells (figure 19)

These data reveal that silencing tenascin-C in RA synovial fibroblasts reduces the synthesis of pro-inflammatory cytokines and suggest that ablation of tenascin-C expression is a viable strategy to inhibit inflammation in the synovium.

This work has established that blocking tenascin-C activity (with peptides) and tenascin-C expression (with siRNA) reduces inflammatory cytokine synthesis in human RA synovia. These data shows that tenascin-C blockade is of potential clinical benefit in treating RA and other inflammatory diseases.

### References

1. Smolen, J.S. & Maini, R.N. Interleukin-6: a new therapeutic target. Arthritis Res Ther 8 Suppl 2, S5 (2006).
2. Williams, R.O., Paleolog, E. & Feldmann, M. Cytokine inhibitors in rheumatoid arthritis and other autoimmune diseases, Curr Opin Pharmacol 7, 412-417 (2007).
3. Brentano, F., Kyburz, D., Schorr, O., Gay, R. & Gay, S. The role of Toll-like receptor signalling in the pathogenesis of arthritis. Cell Immunol 233, 90-96 (2005).
4. O'Neill, L.A. Primer: Toll-like receptor signaling pathways-what do rheumatologists need to know? Nat Clin Pract Rheumatol (2008).
5. Matzinger, P. The danger model: a renewed sense of self. Science 296, 301-305 (2002).
6. Bianchi, M.E. DAMPs, PAMPs and alarmins: all we need to know about danger. J Leukoc Biol 81, 1-5 (2007).
7. Gordon, S. Pattern recognition receptors: doubling up for the innate immune response. Cell 111, 927-930 (2002).
8. Medzhitov, R. & Janeway, C.A., Jr. Decoding the patterns of self and nonself by the innate immune system. Science 296, 298-300 (2002).
9. Radstake, T.R., et al. Expression of toll-like receptors 2 and 4 in rheumatoid synovial tissue and regulation by proinflammatory cytokines interleukin-12 and interleukin-18 via interferon-gamma. Arthritis Rheum 50, 3856-3865 (2004).
10. Roelofs, M.F., et al. The expression of toll-like receptors 3 and 7 in rheumatoid arthritis synovium is increased and costimulation of toll-like receptors 3, 4, and 7/8 results in synergistic cytokine production by dendritic cells. Arthritis Rheum 52, 2313-2322 (2005).
11. Sacre, S.M., et al. The Toll-like receptor adaptor proteins MyD88 and MaI/TIRAP contribute to the inflammatory and destructive processes in a human model of rheumatoid arthritis. Am J Pathol 170, 518-525 (2007).
12. Choe, J.Y., Crain, B., Wu, S.R. & Corr, M. Interleukin 1 receptor dependence of serum transferred arthritis can be circumvented by toll-like receptor 4 signaling. J Exp Med 197, 537-542 (2003).
13. Lee, E.K., Kang, S.M., Paik, D.J., Kim, J.M. & Youn, J. Essential roles of Toll-like receptor-4 signaling in arthritis induced by type II collagen antibody and LPS. Int Immunol 17, 325-333 (2005).
14. Abdollahi-Roodsaz, S., et al. Inhibition of Toll-like receptor 4 breaks the inflammatory loop in autoimmune destructive arthritis. Arthritis Rheum 56, 2957-2967 (2007).
15. Vanags, D., et al. Therapeutic efficacy and safety of chaperonin 10 in patients with rheumatoid arthritis: a double-blind randomised trial. Lancet 368, 855-863 (2006).
16. Chiquet-Ehrismann, R. & Chiquet, M. Tenascins: regulation and putative functions during pathological stress. J Pathol 200, 488-499 (2003).
17. Cutolo, M., Picasso, M., Ponassi, M., Sun, M.Z. & Balza, E. Tenascin and fibronectin distribution in human normal and pathological synovium. J Rheumatol 19, 1439-1447 (1992).
18. McCachren, S.S. & Lightner, V.A. Expression of human tenascin in synovitis and its regulation by interleukin-1. Arthritis Rheum 35, 1185-1196 (1992).
19. Salter, D.M. Tenascin is increased in cartilage and synovium from arthritic knees. Br J Rheumatol 32, 780-786 (1993).
20. Chevalier, X., Groult, N., Larget-Piet, B., Zardi, L. & Hornebeck, W. Tenascin distribution in articular cartilage from normal subjects and from patients with osteoarthritis and rheumatoid arthritis. Arthritis Rheum 37, 1013-1022 (1994).
21. Hasegawa, M., et al. Expression of large tenascin-C splice variants in synovial fluid of patients with rheumatoid arthritis. J Orthop Res 25, 563-568 (2007).
22. Orend, G. Potential oncogenic action of tenascin-C in tumorigenesis. Int J Biochem Cell Biol 37, 1066-1083 (2005).
23. Brackertz, D., Mitchell, G.F. & Mackay, I.R. Antigen-induced arthritis in mice. I. Induction of arthritis in various strains of mice. Arthritis Rheum 20, 841-850 (1977).
24. Brennan, F.M., Chantry, D., Jackson, A., Maini, R. & Feldmann, M. Inhibitory effect of TNF alpha antibodies on synovial cell interleukin-1 production in rheumatoid arthritis. Lancet 2, 244-247 (1989).
25. Smiley, S.T., King, J.A. & Hancock, W.W. Fibrinogen stimulates macrophage chemokine secretion through toll-like receptor 4. J Immunol 167, 2887-2894 (2001).
26. Fitzgerald, K.A., Rowe, D.C. & Golenbock, D.T. Endotoxin recognition and signal transduction by the TLR4/MD2-complex. Microbes Infect 6, 1361-1367 (2004).
27. Jiang, Z., et al. CD14 is required for MyD88-independent LPS signaling. Nat Immunol 6, 565-570 (2005).
28. Coats, S.R., Do, C.T., Karimi-Naser, L.M., Braham, P.H. & Darveau, R.P. Antagonistic lipopolysaccharides block E. coli lipopolysaccharide function at human TLR4 via interaction with the human MD-2 lipopolysaccharide binding site. Cell Microbiol 9, 1191-1202 (2007).
29. Siri, A., et al. Human tenascin: primary structure, pre-mRNA splicing patterns and localization of the epitopes recognized by two monoclonal antibodies. Nucleic Acids Res 19, 525-531 (1991).
30. Gondokaryono, S.P., et al. The extra domain A of fibronectin stimulates murine mast cells via toll-like receptor 4. J Leukoc Biol 82, 657-665 (2007).
31. Taylor, K.R., et al. Recognition of hyaluronan released in sterile injury involves a unique receptor complex dependent on Toll-like receptor 4, CD44, and MD-2. J Biol Chem 282, 18265-18275 (2007).
32. Kim, H.M., et al. Crystal structure of the TLR4-MD-2 complex with bound endotoxin antagonist Eritoran. Cell 130, 906-917 (2007).
33. Schaefer, L., et al. The matrix component biglycan is proinflammatory and signals through Toll-like receptors 4 and 2 in macrophages. J Clin Invest 115, 2223-2233 (2005).
34. Foell, D., Wittkowski, H. & Roth, J. Mechanisms of disease: a 'DAMP' view of inflammatory arthritis. Nat Clin Pract Rheumatol 3, 382-390 (2007).
35. Taniguchi, N., et al. High mobility group box chromosomal protein 1 plays a role in the pathogenesis of rheumatoid arthritis as a novel cytokine. Arthritis Rheum 48, 971-981 (2003).
36. Pullerits, R., et al. High mobility group box chromosomal protein 1, a DNA binding cytokine, induces arthritis. Arthritis Rheum 48, 1693-1700 (2003).
37. Kokkola, R., et al. Successful treatment of collagen-induced arthritis in mice and rats by targeting extracellular high mobility group box chromosomal protein 1 activity. Arthritis Rheum 48, 2052-2058 (2003).
38. Gutowski, N.J., Newcombe, J. & Cuzner, M.L. Tenascin-R and C in multiple sclerosis lesions: relevance to extracellular matrix remodelling. Neuropathol Appl Neurobiol 25, 207-214 (1999).
39. Amin, K., et al. Inflammation and structural changes in the airways of patients with primary Sjogren's syndrome. Respir Med 95, 904-910 (2001).
40. Loots, M.A., et al. Differences in cellular infiltrate and extracellular matrix of chronic diabetic and venous ulcers versus acute wounds. J Invest Dermatol 111, 850-857 (1998).
41. Lange, K., et al. Endothelin receptor type B counteracts tenascin-C-induced endothelin receptor type A-dependent focal adhesion and actin stress fiber disorganization. Cancer Res 67, 6163-6173 (2007).
42. Saga, Y., Yagi, T., Ikawa, Y., Sakakura, T. & Aizawa, S. Mice develop normally without tenascin. Genes Dev 6, 1821-1831 (1992).
43. Hoshino, K., et al. Cutting edge: Toll-like receptor 4 (TLR4)-deficient mice are hyporesponsive to lipopolysaccharide: evidence for TLR4 as the Lps gene product. J Immunol 162, 3749-3752 (1999).
44. Takeuchi, O., et al. Differential roles of TLR2 and TLR4 in recognition of gram-negative and gram-positive bacterial cell wall components. Immunity 11, 443-451 (1999).
45. Keystone, E.C., Schorlemmer, H.U., Pope, C. & Allison, A.C. Zymosan-induced arthritis: a model of chronic proliferative arthritis following activation of the alternative pathway of complement. Arthritis Rheum 20, 1396-1401 (1977).
46. van Lent, P.L., et al. Fcgamma receptors directly mediate cartilage, but not bone, destruction in murine antigen-induced arthritis: uncoupling of cartilage damage from bone erosion and joint inflammation. Arthritis Rheum 54, 3868-3877 (2006).
47. Foxwell, B., et al. Efficient adenoviral infection with IkappaB alpha reveals that macrophage tumor necrosis factor alpha production in rheumatoid arthritis is NF-kappaB dependent. Proc Natl Acad Sci U S A 95, 8211-8215 (1998).
48. Kurt-Jones, E.A., et al. Use of murine embryonic fibroblasts to define Toll-like receptor activation and specificity. J Endotoxin Res 10, 419-424 (2004).
49. Todaro, G.J. & Green, H. Quantitative studies of the growth of mouse embryo cells in culture and their development into established lines. J Cell Biol 17, 299-313 (1963).
50. Butler, D.M., Malfait, A.M., Maini, R.N., Brennan, F.M. & Feldmann, M. Anti-IL-12 and anti-TNF antibodies synergistically suppress the progression of murine collagen-induced arthritis. Eur J Immunol 29, 2205-2212 (1999).
51. Ho, S.N., Hunt, H.D., Horton, R.M., Pullen, J.K. & Pease, L.R. Site-directed mutagenesis by overlap extension using the polymerase chain reaction. Gene 77, 51-59 (1989).
52. Clark, R.A., Erickson, H.P. & Springer, T.A. Tenascin supports lymphocyte rolling. J Cell Biol 137, 755-765 (1997).
53. El-Karef, A., et al. Deficiency of tenascin-C attenuates liver fibrosis in immune-mediated chronic hepatitis in mice. J Pathol 211, 86-94 (2007).
54. Loike, J.D., Cao, L., Budhu, S., Hoffman, S. & Silverstein, S.C. Blockade of alpha 5 beta 1 integrins reverses the inhibitory effect of tenascin on chemotaxis of human monocytes and polymorphonuclear leukocytes through three-dimensional gels of extracellular matrix proteins. J Immunol 166, 7534-7542 (2001).
55. Talts, J.F., Wirl, G., Dictor, M., Muller, W.J. & Fassler, R. tenascin-C modulates tumor stroma and monocyte/macrophage recruitment but not tumor growth or metastasis in a mouse strain with spontaneous mammary cancer. J Cell Sci 112 (Pt 12), 1855-1864 (1999).
56. Jones (2000) Matrix Biol.,19, 581-96
57. Harandl (2009) Expert Review of Vaccines, 8, 293-298
58. McIntyre (2006) BMC Biotechnol. 6: 1
59. Paddison (2002) Genes Dev. 16 (8): 948-58
60. Andreakos (2004) Blood, 103, 2229-37
61. Goh, F.G., Piccinini, A.M., Krausgruber, T., Udalova, I.A. & Midwood, K.S. Transcriptional regulation of the endogenous danger signal tenascin-C: a novel autocrine loop in inflammation. J Immunol 184, 2655-2662 (2010).
62. Midwood, K. et al. Tenascin-C is an endogenous activator of Toll-like receptor 4 that is essential for maintaining inflammation in arthritic joint disease. Nat Med 15, 774-780 (2009).
63. LaFleur, D.W. et al. Aortic smooth muscle cells interact with tenascin-C through its fibrinogen-like domain. J Biol Chem 272, 32798-32803 (1997).
64. Taylor, P.C. & Feldmann, M. Anti-TNF biologic agents: still the therapy of choice for rheumatoid arthritis. Nat Rev Rheumatol 5, 578-582 (2009).
65. Yokoyama, K., Erickson, H.P., Ikeda, Y. & Takada, Y. Identification of amino acid sequences in fibrinogen gamma -chain and tenascin C C-terminal domains critical for binding to integrin alpha vbeta 3. J Biol Chem 275, 16891-16898 (2000).

## Claims

1. An agent for modulation of a chronic inflammatory response wherein the agent modulates the biological activity of tenascin-C, wherein the agent is an antibody or antigen-binding fragment thereof having specificity for the FBG domain of tenascin-C.

2. An agent as claimed in claim 1 wherein the agent modulates the biological activity of tenascin-C by altering the binding properties of tenascin-C.

3. An agent as claimed in any of claims 1-2 wherein the agent is an inhibitor of the binding properties of tenascin-C, or wherein the agent is a competive binding inhibitor of tenascin-C.

4. An agent as claimed in any one of claims 1-3 wherein the antibody or antigen-binding fragment thereof is selected from the group consisting of Fv fragments, scFv fragments, Fab, single variable domains and domain antibodies, and wherein the antibody or antigen-binding fragment thereof is optionally humanised.

5. An agent claimed in any of claims 1-4 wherein the chronic inflammatory response is associated with a condition **characterised by** inappropriate inflammation, for example wherein the chronic inflammatory response is associated with rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

6. A composition comprising an agent as defined in any of claims 1-5 and a pharmaceutically acceptable carrier, excipient and/or diluent, and optionally further comprising at least one other agent.

7. A composition as claimed in claim 6 wherein the at least one other agent is an anti-inflammatory agent (for example non-steroidal anti-inflammatories (NSAIDs), corticosteroids, disease-modifying antirheumatic drugs (DMARDs) or immunosuppressants, a statin, a biological agent (biologicals), an immunosuppressive agent, a salicylate and/or a microbicidal agent.

8. An agent or composition as defined in claims 1-7 for use in the treatment of a chronic inflammatory condition, wherein the chronic inflammatory response is associated with rheumatoid arthritis (RA), inflammatory bowel diseases, atherosclerosis and/or psoriasis.

9. Use of an agent or composition as defined in claims 1-7 in the manufacture of a medicament for the treatment of a chronic inflammatory condition, wherein the chronic inflammatory response is associated with rheumatoid arthritis (RA), inflammatory bowel diseases, atherosclerosis and/or psoriasis.

10. A kit of parts comprising:
(i) an agent or composition as defined in claims 1-7
(ii) administration means
(iii) instructions for their use
and optionally further comprising
(iv) at least one other agent.

## Patentansprüche

1. Mittel zur Modulation einer chronischen entzündlichen Reaktion, wobei das Mittel die biologische Aktivität von Tenascin-C moduliert, wobei das Mittel ein Antikörper oder ein antigenbindendes Fragment von ihm mit Spezifität für die FBG-Domäne von Tenascin-C ist.

2. Mittel, wie es im Anspruch 1 beansprucht wird, wobei das Mittel die biologische Aktivität von Tenascin-C über die Veränderung der Bindungseigenschaften von Tenascin-C moduliert.

3. Mittel, wie es in irgendeinem der Ansprüche 1-2 beansprucht wird, wobei das Mittel ein Inhibitor der Bindungseigenschaften von Tenascin-C ist oder wobei das Mittel ein kompetitiver Bindingsinhibitor von Tenascin-C ist.

4. Mittel, wie es in irgendeinem der Ansprüche 1-3 beansprucht wird, wobei der Antikörper oder das antigenbindende Fragment von ihm aus der Gruppe ausgewählt ist, die aus Fv-Fragmenten, scFv-Fragmenten, Fab, einzelnen variablen Domänen und Domänenantikörpern besteht, und wobei der Antikörper oder das antigenbindende Fragment von ihm gegebenenfalls humanisiert ist.

5. Mittel, wie es in irgendeinem der Ansprüche 1-4 beansprucht wird, wobei die chronische entzündliche Reaktion mit einer Erkrankung assoziiert ist, die durch eine unerwünschte Entzündung gekennzeichnet ist, zum Beispiel wobei die chronische entzündliche Reaktion assoziiert ist mit rheumatoider Arthritis (RA), Autoimmunerkrankungen, entzündlichen Darmerkrankungen, nicht heilenden Wunden, multipler Sklerose, Krebs, Atherosklerose, dem Sjögren-Syndrom, Diabetes, Lupus erythematodes (einschließlich systemischer Lupus erythematodes), Asthma, fibrotischen Erkrankungen (einschließlich Leberzirrhose), Lungenfibrose, UV-Schäden und Psoriasis.

6. Zusammensetzung, die ein Mittel, wie es in irgendeinem der Ansprüche 1-5 definiert ist, und einen pharmazeutisch annehmbaren Träger, Hilfsstoff und/oder ein pharmazeutisch annehmbares Verdünnungsmittel umfasst und gegebenenfalls ferner wenigstens ein weiteres Mittel umfasst.

7. Zusammensetzung, wie sie im Anspruch 6 beansprucht wird, wobei das wenigstens eine weitere Mittel ein Entzündungshemmer ist (zum Beispiel nichtsteroidale Entzündungshemmer (NSAIDs), Corticosteroide, Disease Modifying Antirheumatic Drugs (DMARDs) oder Immunsuppressiva, ein Statin, ein biologisches Mittel (Biologika), ein immunsuppressives Mittel, ein Salicylat und/oder ein mikrobizides Mittel.

8. Mittel oder Zusammensetzung, wie es bzw. sie in den Ansprüchen 1-7 definiert ist, zur Verwendung bei der Behandlung einer chronischen entzündlichen Erkrankung, wobei die chronische entzündliche Reaktion mit rheumatoider Arthritis (RA), entzündlichen Darmerkrankungen, Atherosklerose und/oder Psoriasis assoziiert ist.

9. Verwendung eines Mittels oder einer Zusammensetzung, wie es bzw. sie in den Ansprüchen 1-7 definiert ist, bei der Herstellung eines Medikaments für die Behandlung einer chronischen entzündlichen Erkrankung, wobei die chronische entzündliche Reaktion mit rheumatoider Arthritis (RA), entzündlichen Darmerkrankungen, Atherosklerose und/oder Psoriasis assoziiert ist.

10. Kit aus Teilen, das umfasst:
(i) Mittel oder Zusammensetzung, wie es bzw. sie in den Ansprüchen 1-7 definiert ist
(ii) Vorrichtung zur Verabreichung
(iii) Anleitungen für ihren Gebrauch
und das gegebenenfalls ferner umfasst
(iv) wenigstens ein weiteres Mittel.

## Revendications

1. Agent de modulation d'une réponse inflammatoire chronique dans lequel l'agent module l'activité biologique de la ténascine-C, dans lequel l'agent est un anticorps ou un fragment de liaison à l'antigène de celui-ci ayant une spécificité pour le domaine FBG de la ténascine-C.

2. Agent selon la revendication 1, dans lequel l'agent module l'activité biologique de la ténascine-C en modifiant les propriétés de liaison de la ténascine-C.

3. Agent selon l'une quelconque des revendications 1 à 2, dans lequel l'agent est un inhibiteur des propriétés de liaison de la ténascine-C, ou dans lequel l'agent est un inhibiteur de liaison compétitif de la ténascine-C.

4. Agent selon l'une quelconque des revendications 1 à 3, dans lequel l'anticorps ou le fragment de liaison à l'antigène de celui-ci est choisi dans le groupe constitué par les fragments Fv, les fragments scFv, Fab, des domaines variables uniques et les anticorps de domaine, et dans lequel l'anticorps ou le fragment de liaison à l'antigène de celui-ci est éventuellement humanisé.

5. Agent selon l'une quelconque des revendications 1 à 4, dans lequel la réponse inflammatoire chronique est associée à une affection **caractérisée par** une inflammation inopportune, par exemple dans lequel la réponse inflammatoire chronique est associée à la polyarthrite rhumatoïde (PR), les affections auto-immunes, les maladies inflammatoires chroniques de l'intestin, les plaies ne cicatrisant pas, la sclérose en plaques, le cancer, l'athérosclérose, la maladie de Sjogrens, le diabète, le lupus érythémateux (y compris le lupus érythémateux disséminé), l'asthme, les maladies fibreuses (y compris la cirrhose du foie), la fibrose pulmonaire, les effets nocifs des UV et le psoriasis.

6. Composition comprenant un agent tel que défini dans l'une quelconque des revendications 1 à 5 et vecteur, excipient et/ou diluant pharmaceutiquement acceptables, et éventuellement comprenant en outre au moins un autre agent.

7. Composition selon la revendication 6, dans lequel le au moins un autre agent est un agent anti-inflammatoire (par exemple, des anti-inflammatoires non stéroïdiens (AINS), des corticostéroïdes, les médicaments antirhumatismaux modificateurs de la maladie (ARMN) ou les immunosuppresseurs, une statine, un agent biologique (produits biologiques), un agent immunosuppresseur, un salicylate et/ou un agent microbicide.

8. Agent ou composition tel(le) que défini (e) dans les revendications 1 à 7 pour utilisation dans le traitement d'une affection inflammatoire chronique, dans lequel/laquelle la réponse inflammatoire chronique est associée à la polyarthrite rhumatoïde (PR), les maladies inflammatoires chroniques de l'intestin, l'athérosclérose et/ou le psoriasis.

9. Utilisation d'un agent ou d'une composition tel(le)s que défini€s dans les revendications 1 à 7 dans la fabrication d'un médicament destiné au traitement d'une affection inflammatoire chronique, dans laquelle la réponse inflammatoire chronique est associée à la polyarthrite rhumatoïde (PR), les maladies inflammatoires chroniques de l'intestin, l'athérosclérose et/ou le psoriasis.

10. Kit d'éléments comprenant :
(i) un agent ou une composition tel (le) que défini(e) dans les revendications 1 à 7
(ii) un moyen d'administration
(iii) des instructions pour leur utilisation
et éventuellement comprenant en outre
(iv) au moins un autre agent.
